# EUROPEAN PATENT SPECIFICATION

(11) **EP 3 897 550 B1**
(45) Date of publication and mention of the grant of the patent: **16.07.2025**
(21) Application number: 19832672.0
(22) Date of filing: 19.12.2019
(51) Int. Cl.: A61K 8/34, A61Q 5/06, A61K 8/81, A61K 8/892, A61K 8/04, A61K 8/19

(54) **AEROSOL DEVICE CONTAINING A COSMETIC COMPOSITION COMPRISING A FIXING POLYMER, A HYDROXYLATED SILICONE AND A POWDER**
AEROSOLVORRICHTUNG MIT EINER KOSMETISCHEN ZUSAMMENSETZUNG MIT EINEM FIXIERENDEN POLYMER, EINEM HYDROXYLIERTEN SILIKON UND EINEM PULVER
DISPOSITIF AÉROSOL CONTENANT UNE COMPOSITION COSMÉTIQUE RENFERMANT UN POLYMÈRE FIXANT, UNE SILICONE HYDROXYLÉE ET UNE POUDRE

(30) Priority: 20.12.2018 FR 1873654
(43) Date of publication of application: 27.10.2021
(73) Proprietor: L'OREAL, 75008 Paris (FR)
(72) Inventor: GAWTREY, Jonathan, 93400 SAINT-OUEN (FR); AUBERT, Lionel, 93400 SAINT-OUEN (FR); SMAIL, Nadia, 93400 SAINT-OUEN (FR)
(74) Representative: Casalonga
(86) International application number: PCT/EP2019/086464
(87) International publication number: WO 2020/127835

(56) References cited:
- WO-A1-2014/177649
- WO-A1-2018/162711
- DATABASE GNPD [online] MINTEL; 17 September 2004 (2004-09-17), ANONYMOUS: "Improved Silk & Soy Mousse", XP055607233, retrieved from www.gnpd.com Database accession no. 10187794
- DATABASE GNPD [online] MINTEL; 1 February 2016 (2016-02-01), ANONYMOUS: "Volumizing Foam", XP055607230, retrieved from www.gnpd.com Database accession no. 3762589

## Description

The invention relates to an aerosol device containing a cosmetic composition comprising at least one fixing polymer, at least one specific hydroxylated silicone, at least one powder and at least one propellant.

The present invention also relates to a method for the cosmetic treatment of keratinous fibres, in particular human keratinous fibres, such as the hair, comprising the application, to the said keratinous fibres, of the said cosmetic composition sprayed from the said aerosol device.

The invention additionally relates to the use of the said cosmetic composition sprayed from the said aerosol device for the styling, that is to say for the shaping of the fibres and/or the form retention of the hairstyle, and/or the colouring of keratinous fibres, in particular human keratinous fibres, such as the hair.

Styling products are normally used to construct and structure the hairstyle and to give it hold. They are usually provided in the form of spray compositions, such as lacquers or sprays. These compositions generally comprise one or more film-forming polymers, or "fixing polymers", which make possible the formation of a sheathing film on the fibres and/or the formation of microwelds between the fibres, thus ensuring the form retention of the hairstyle and the fixing of the head of hair.

WO2018/162711 discloses an aerosol device comprising polyvinylcaprolactam, calcium carbonate, dimethiconol, ethanol and butane.

These compositions are generally applied to wet hair, which is shaped before carrying out blow drying or drying, or to dry hair, in finishing, after the shaping.

In order to obtain a satisfactory and long-lasting fixing power, it is known practice to incorporate, in styling products, polymers having a high fixing power and/or to increase the concentration of fixing polymer. However, the use of such products results in a certain number of disadvantages.

Although the objective of these products is to ensure the fixing and the hold of the hairstyle over time, they generally have a tendency to make the hairstyle rigid, in particular producing a "helmet effect", often badly received by users. The head of hair, thus made rigid, exhibits a dry and rough feel which is not greatly appreciated by consumers.

Furthermore, when the composition is provided in the spray form, and in particular in the dry, that is to say non-wetting, spray form, large amounts of propellants are necessary in order to obtain satisfactory spraying of the composition over the fibres. However, the use of such amounts of propellants results in the formation of very fine particles (with a mean diameter of less than 10 µm) which can be inhaled by the consumer during the application of the composition. The inhalation of these particles can then result in coughing and irritation, admittedly transitory but nevertheless unpleasant for the consumer.

Some of these compositions can also, in addition, comprise colouring powders, such as pigments or pearlescent agents. These make-up compositions make it possible to deposit colouring and/or pearlescent agents at the surface without threatening the integrity of the fibres and can thus be used to temporarily colour keratinous fibres. In point of fact, compositions of this type generally exhibit the disadvantage of transferring the colouring particles onto the surfaces with which they are brought into contact, such as the hands, the scalp and the clothes of the consumers.

Thus, there exists a real need to make available a composition in the spray form which does not exhibit the abovementioned disadvantages, i.e. which limits, indeed even prevents, the formation of fine particles and which makes it possible to obtain a long-lasting fixing of the hairstyle, with styling effects which persist throughout the day, while conferring a natural and non-rigid appearance on the hairstyle. Furthermore, there exists a need to make available compositions for making up the hair in the spray form which limit, indeed even prevent, the transfer of the colouring particles.

It has been discovered, surprisingly, that the combination, within an aerosol device, of at least one fixing polymer, of at least one specific hydroxylated silicone, of at least one powder, of at least one organic solvent and of dimethyl ether makes it possible to achieve the objectives set out above, in particular to obtain a composition capable of conferring volume and a long-lasting fixing on keratinous fibres while retaining a natural and non-rigid appearance, and also a full-of-body feel, and optionally of colouring keratinous fibres.

The composition of the invention additionally makes it possible to limit the amount of fine particles and thus to be less irritating to consumers.

Moreover, the composition of the invention exhibits optimum spray qualities, in particular a gentle spray which does not ruffle the hairstyle and without blocking.

A subject matter of the present invention is in particular an aerosol device containing a cosmetic composition comprising:
- one or more fixing polymers,
- one or more powders,
- at least 0.05% by weight, with respect to the total weight of the composition, of one or more polydimethylsiloxanes comprising dimethylsilanol end groups,
- one or more organic solvents, and
- one or more propellants, at least one of which is dimethyl ether;
the ratio by weight of the total amount of the powder(s) to the total amount of the fixing polymer(s) is greater than or equal to 1.

The composition according to the invention confers flexibility and volume on the hair, while retaining a natural appearance and a full-of-body feel. The head of hair styled using the composition of the invention is form-retained without being set rigid and the styling effects which are conferred on it persist throughout the day.

According to a specific embodiment of the invention, the cosmetic composition contained in the aerosol device comprises:
- one or more fixing polymers,
- one or more powders, a least one of which is chosen from pigments and their mixtures,
- at least 0.05% by weight, with respect to the total weight of the composition, of one or more polydimethylsiloxanes comprising dimethylsilanol end groups,
- one or more organic solvents, and
- one or more propellants, at least one of which is dimethyl ether;
the ratio by weight of the total amount of the powder(s) to the total amount of the fixing polymer(s) is greater than or equal to 1.

The sprayed composition according to this embodiment makes it possible to make up the hair without, however, resulting in transfer of colouring onto the hands, the scalp or the clothes of the consumers. In addition, it confers good colouring and fixing properties on the hair, while being more pleasant to use for the consumer.

Another subject-matter of the present invention is a method for the cosmetic treatment, in particular the shaping and/or the colouring, of keratinous fibres, in particular human keratinous fibres, such as the hair, comprising a step of application, to the said keratinous fibres, of a cosmetic composition sprayed from an aerosol device as defined above.

Another subject-matter of the present invention is the use of a cosmetic composition sprayed from an aerosol device as defined above, for the styling, that is to say for the shaping of the fibres and/or the form retention of the hairstyle, and/or the colouring of keratinous fibres, in particular human keratinous fibres, such as the hair.

Other subject-matters, characteristics, aspects and advantages of the invention will become even more clearly apparent on reading the description and the examples which follow.

In that which will follow, and unless otherwise indicated, the limits of a range of values are included in this range, in particular in the expressions "of between" and "ranging from ... to ...".

The expression "at least one" used in the present description is equivalent to the expression "one or more".

### The fixing polymers

The cosmetic composition contained in the aerosol device according to the invention comprises one or more fixing polymers.

Within the meaning of the present invention, the term "fixing polymer" is understood to mean a polymer which makes possible the fixing of the shaping of keratinous fibres, in particular of the hair.

Preferably, the fixing polymer(s) are chosen from anionic fixing polymers, cationic fixing polymers, amphoteric fixing polymers, non-ionic fixing polymers, and their mixtures.

The anionic fixing polymers which can be used according to the present invention are polymers comprising groups derived from carboxylic, sulfonic or phosphoric acid and have a number-average molecular weight of between approximately 500 and 5 000 000.

The carboxylic groups are provided by unsaturated mono- or dicarboxylic acid monomers, such as those corresponding to the formula (I): 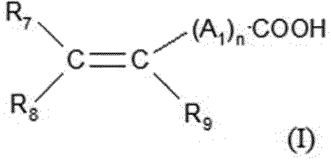 in which n is an integer from 0 to 10, A₁ denotes a methylene group, optionally connected to the carbon atom of the unsaturated group or to the adjacent methylene group when n is greater than 1, via a heteroatom, such as oxygen or sulfur, R₇ denotes a hydrogen atom or a phenyl or benzyl group, R₈ denotes a hydrogen atom or a lower alkyl or carboxyl group, and R₉ denotes a hydrogen atom, a lower alkyl group or a - CH₂-COOH, phenyl or benzyl group.

In the abovementioned formula, a lower alkyl group preferably denotes a group having from 1 to 4 carbon atoms and in particular the methyl and ethyl groups.

The anionic fixing polymers having carboxylic groups which are preferred according to the invention are:
A) Copolymers of acrylic or methacrylic acid or their salts.
   Mention may be made, among these polymers, of copolymers of acrylic or methacrylic acid with a monoethylenic monomer, such as ethylene, styrene, vinyl esters or acrylic or methacrylic acid esters, optionally grafted to a polyalkylene glycol, such as polyethylene glycol, and optionally crosslinked. Such polymers are described especially in French Patent 1 222 944 and German Application No. 2 330 956, the copolymers of this type comprising an optionally N-alkylated and/or N-hydroxyalkylated acrylamide unit in their chain, as are described in particular in Luxembourgian Patent Applications Nos. 75370 and 75371. Mention may also be made of copolymers of acrylic acid and of C₁-C₄ alkyl methacrylate and terpolymers of vinylpyrrolidone, of acrylic acid and of C₁-C₂₀ alkyl methacrylate, for example lauryl methacrylate, such as that sold by ISP under the name Acrylidone^{®} LM (INCI name: VP/acrylates/lauryl methacrylate copolymer), acrylic acid/ethyl acrylate/N-(t-butyl)acrylamide terpolymers, such as the products Ultrahold^{®} Strong and Ultrahold^{®} 8 sold by BASF (INCI name: Acrylates/t-butylacrylamide copolymer), methacrylic acid/ethyl acrylate/*tert*-butyl acrylate terpolymers, such as the products sold under the name Luvimer^{®} 100 P or Luvimer^{®} PRO 55 by BASF (INCI name: Acrylates copolymer), copolymers of methacrylic acid and of ethyl acrylate, such as the products sold under the name Luvimer^{®} MAE or Luviflex^{®} Soft by BASF (INCI name: Acrylates copolymer), acrylic acid/butyl acrylate/methyl methacrylate terpolymers, such as the product sold under the name Balance^{®} CR by AkzoNobel (INCI name: Acrylates copolymer), or the copolymers of methacrylic acid and of methyl methacrylate sold under the name Eudragit^{®} L 100 by Rohm Pharma (INCI name: Acrylates copolymer). Mention may also be made of branched block polymers containing (meth)acrylic acid monomers, such as the product sold under the name Fixate^{®} G-100L by Lubrizol (INCI name: AMP-acrylates/allyl methacrylate copolymer).
B) Crotonic acid copolymers, such as those comprising, in their chain, vinyl acetate or propionate units and optionally other monomers, such as allyl or methallyl esters, vinyl ethers or vinyl esters of a linear or branched saturated carboxylic acid having a long hydrocarbon chain, such as those comprising at least 5 carbon atoms, it optionally being possible for these polymers to be grafted or crosslinked, or also another monomer which is a vinyl, allyl or methallyl ester of an α- or β-cyclic carboxylic acid. Such polymers are described, *inter alia,* in French Patents 1 222 944, 1 580 545, 2 265 782, 2 265 781, 1 564 110 and 2 439 798. Commercial products coming within this category are the products Resyn^{®} 28-2930 and 28-1310 sold by AkzoNobel (INCI names: VA/crotonates/vinyl decanoate copolymer and VA/crotonates copolymer, respectively). Mention may also be made of the products Luviset^{®} CA 66, sold by BASF, Aristoflex^{®} A60, sold by Clariant (INCI name: VA/crotonates copolymer), and Mexomere^{®} PW or PAM, sold by Chimex (INCI name: VA/vinyl butylbenzoate/crotonates copolymer).
C) Copolymers of monounsaturated C₄-C₈ carboxylic acids or anhydrides chosen from:
   - copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and its esters, the anhydride functional groups of these copolymers optionally being monoesterified or monoamidated. Such polymers are described in particular in US Patents 2 047 398, 2 723 248 and 2 102 113, and GB Patent 839 805. Commercial products are in particular those sold under the names Gantrez^{®} AN or ES by ISP, such as Gantrez^{®} ES 225 (INCI name: Ethyl ester of PVM/MA copolymer) or Gantrez^{®} ES 425L (INCI name: Butyl ester of PVM/MA copolymer).
   - copolymers comprising (i) one or more maleic, citraconic or itaconic anhydride units and (ii) one or more monomers chosen from allyl or methallyl esters, optionally comprising one or more acrylamide, methacrylamide, α-olefin, acrylic or methacrylic ester, acrylic or methacrylic acid or vinylpyrrolidone groups in their chain,
      the anhydride functional groups of these copolymers optionally being monoesterified or monoamidated.
      These polymers are, for example, described in Patents FR 2 350 384 and FR 2 357 241.
D) Polyacrylamides comprising carboxylate groups.

The fixing polymers having units derived from sulfonic acid can be chosen from:
A') Homopolymers and copolymers comprising vinylsulfonic, styrenesulfonic, naphthalenesulfonic or acrylamidoalkylsulfonic units.
   These polymers can in particular be chosen from:
   - salts of polyvinylsulfonic acid having a molecular weight of between approximately 1000 and 100 000, as well as copolymers with an unsaturated comonomer, such as acrylic or methacrylic acids and their esters, as well as acrylamide or its derivatives, vinyl ethers and vinylpyrrolidone;
   - salts of polystyrenesulfonic acid, such as the sodium salts which are sold, for example under the name Flexan^{®} II, by AkzoNobel (INCI name: Sodium polystyrene sulfonate). These compounds are described in Patent FR 2 198 719;
   - salts of polyacrylamidesulfonic acids, such as those mentioned in Patent US 4 128 631, and more particularly the polyacrylamidoethylpropanesulfonic acid sold under the name Rheocare^{®} HSP-1180 by Cognis (INCI name: polyacrylamidomethylpropane sulfonic acid).
B') sulfonic polyesters, these polymers being advantageously obtained by polycondensation of at least one dicarboxylic acid, of at least one diol or of a mixture of diol and of diamine, and of at least one difunctional monomer comprising a sulfonic functional group. Mention may be made, among these polymers, of:
   - linear sulfonic polyesters, such as those described in Patent Applications US 3 734 874, US 3 779 993, US 4 119 680, US 4 300 580, US 4 973 656, US 5 660 816, US 5 662 893 and US 5 674 479. Such polymers are, for example, the products Eastman^{®} AQ38S Polymer, Eastman^{®} AQ55S Polymer and Eastman^{®} AQ48 Ultra Polymer sold by Eastman Chemical (name: Polyester-5) which are copolymers obtained from diethylene glycol, from 1,4-cyclohexanedimethanol, from isophthalic acid and from salt of sulfoisophthalic acid.
   - branched sulfonic polyesters, such as those described in Patent Applications WO 95/18191, WO 97/08261 and WO 97/20899. Such compounds are, for example, the products Eastman^{®} AQ10D Polymer (name: Polyester-13) or Eastman^{®} AQ1350 Polymer which are provided by Eastman Chemical (name: Polyester-13).

According to the invention, the anionic fixing polymers are preferably chosen from copolymers of acrylic acid, such as acrylic acid/ethyl acrylate/N-(tert-butyl)acrylamide terpolymers, sold in particular under the name Ultrahold^{®} Strong by BASF, copolymers derived from crotonic acid, such as vinyl acetate/vinyl *tert-*butylbenzoate/crotonic acid terpolymers and crotonic acid/vinyl acetate/vinyl neododecanoate terpolymers, sold in particular under the name Resyn 28-2930 by AkzoNobel, polymers derived from maleic, fumaric or itaconic acids or anhydrides with vinyl esters, vinyl ethers, vinyl halides, phenylvinyl derivatives and acrylic acid and its esters, such as methyl vinyl ether/monoesterified maleic anhydride copolymers, sold, for example, under the names Gantrez^{®} ES 425L or ES 225 by ISP, copolymers of methacrylic acid and of ethyl acrylate sold under the name Luvimer^{®} MAE by BASF, and vinyl acetate/crotonic acid copolymers sold under the name Luviset^{®} CA 66 by BASF, and vinyl acetate/crotonic acid copolymers grafted with polyethylene glycol sold under the name Aristoflex^{®} A60 by Clariant, vinylpyrrolidone/acrylic acid/lauryl methacrylate terpolymers sold under the name Acrylidone^{®} LM by ISP, the polymer sold under the name Fixate^{®} G-100L by Lubrizol, vinyl acetate/crotonic acid/vinyl p-tert-butylbenzoate copolymers sold under the names Mexomere^{®} PW or PAM by Chimex.

The cationic fixing polymers which can be used according to the present invention are preferably chosen from polymers comprising primary, secondary, tertiary and/or quaternary amine groups forming part of the polymer chain or directly connected to the latter, and having a molecular weight of between 500 and approximately 5 000 000 and preferably between 1000 and 3 000 000.

Mention may more particularly be made, among these polymers, of the following cationic polymers:
(1) Homopolymers or copolymers derived from acrylic or methacrylic esters or amides and comprising at least one of the units of following formulae: 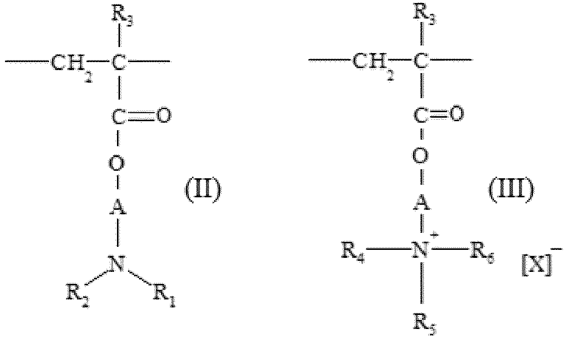 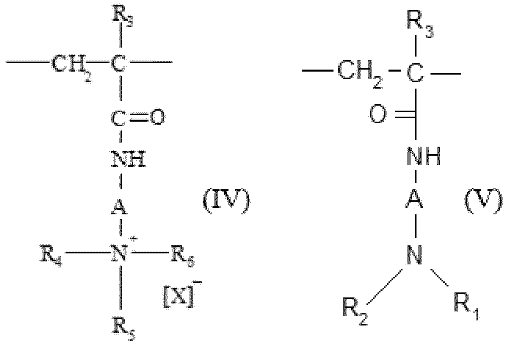 in which:
   - R₃ denotes a hydrogen atom or a CH₃ radical;
   - A is a linear or branched alkyl group comprising from 1 to 6 carbon atoms or a hydroxyalkyl group comprising from 1 to 4 carbon atoms;
   - R₄, R₅ and R₆, which are identical or different, represent an alkyl group having from 1 to 18 carbon atoms or a benzyl radical;
   - R₁ and R₂, which are identical or different, each represent a hydrogen atom or an alkyl group having from 1 to 6 carbon atoms; and
   - X denotes a methosulfate anion or a halide, such as chloride or bromide.
   The copolymers of the family (1) additionally comprise one or more units deriving from comonomers which can be chosen from the family of the acrylamides, methacrylamides, diacetone acrylamides, acrylamides and methacrylamides substituted on the nitrogen by lower (C₁-C₄) alkyl groups, groups derived from acrylic or methacrylic acids or their esters, vinyllactams, such as vinylpyrrolidone or vinylcaprolactam, or vinyl esters.
   Thus, mention may be made, among these copolymers of the family (1), of:
   - vinylpyrrolidone/dialkylaminoalkyl acrylate or methacrylate copolymers, which may or may not be quaternized, such as the products sold under the Gafquat^{®} name by ISP, such as, for example, Gafquat^{®} 734 or Gafquat^{®} 755 or Gafquat^{®} 755N (INCI name: Polyquaternium-11), or alternatively the products known as Copolymer^{®} 845, 958 and 937 sold by ISP (INCI name: VP/dimethylaminoethyl methacrylate copolymer). These polymers are described in detail in French Patents 2 077 143 and 2 393 573,
   - polymers comprising a fatty chain and comprising a vinylpyrrolidone unit, such as the products sold under the names Styleze^{®} W20L and Styleze^{®} W10 by ISP (INCI name: Polyquaternium-55),
   - dimethylaminoethyl methacrylate/vinylcaprolactam/vinylpyrrolidone terpolymers, such as the products sold under the names Advantage HC 37 or Gaffix^{®} VC 713 by ISP (INCI name: Vinylcaprolactam/VP/dimethylaminoethyl methacrylate copolymer), and
   - and quaternized vinylpyrrolidone/dimethylaminopropylmethacrylamide copolymers, such as the product sold under the name Gafquat^{®} HS 100 by ISP (INCI name: Polyquaternium-28).
(2) Cationic derivatives of guar gum, preferably comprising quaternary ammonium, such as those described in the United States Patents 3 589 578 and 4 031 307, such as guar gums containing trialkylammonium cationic groups. Such products are sold in particular under the trade names of Jaguar^{®} C13 S, Jaguar^{®} C 15 and Jaguar^{®} C 17 by Rhodia (INCI name: Guar hydroxypropyltrimonium chloride).
(3) Quaternary copolymers of vinylpyrrolidone and of vinylimidazole; mention may be made, for example, of vinylpyrrolidone/methylvinylimidazolium chloride copolymers, such as the products sold by BASF under the names Luviquat^{®} FC550 or FC370, Luviquat^{®} Excellence, Luviquat^{®} Style (INCI name: Polyquaternium-16), or of vinylpyrrolidone/vinylimidazolium methosulfate/vinylcaprolactam terpolymers, such as the product Luviquat^{®} Hold sold by BASF (INCI name: Polyquaternium-46).
(4) Chitosans or their salts; the salts which can be used are in particular chitosan acetate, lactate, glutamate, gluconate or pyrrolidonecarboxylate.
   Mention may be made, among these compounds, of the chitosan pyrrolidonecarboxylate sold under the name Kytamer^{®} PC by Amerchol (INCI name: Chitosan PCA).
(5) Cationic cellulose derivatives, such as copolymers of cellulose or of cellulose derivatives grafted with a water-soluble monomer comprising a quaternary ammonium, and described in particular in Patent US 4 131 576, such as hydroxyalkylcelluloses, for example hydroxymethyl-, hydroxyethyl- or hydroxypropylcelluloses, grafted in particular with a methacryloyloxyethyltrimethylammonium, methacrylamidopropyltrimethylammonium or dimethyldiallylammonium salt.

The marketed products corresponding to this definition are more particularly the products sold under the names Celquat^{®} L 200 and Celquat^{®} H 100 by AkzoNobel (INCI name: Polyquaternium-4).

The amphoteric fixing polymers which can be used in accordance with the invention can be chosen from polymers comprising B and C units randomly distributed in the polymer chain, where B denotes a unit deriving from a monomer comprising at least one basic nitrogen atom and C denotes a unit deriving from an acidic monomer comprising one or more carboxylic or sulfonic groups or else B and C can denote groups deriving from zwitterionic carboxybetaine or sulfobetaine monomers;
B and C can also denote a cationic polymer chain comprising primary, secondary, tertiary or quaternary amine groups, in which at least one of the amine groups carries a carboxylic or sulfonic group connected via a hydrocarbon group, or else B and C form part of a chain of a polymer comprising an α,β-dicarboxyethylene unit, one of the carboxylic groups of which has been reacted with a polyamine comprising one or more primary or secondary amine groups.

The more particularly preferred amphoteric fixing polymers corresponding to the definition given above are chosen from the following polymers:
(1) Copolymers comprising acidic vinyl units and comprising basic vinyl units, such as those resulting from the copolymerization of a monomer derived from a vinyl compound carrying a carboxylic group, such as more particularly acrylic acid, methacrylic acid, maleic acid or α-chloroacrylic acid, and of a basic monomer derived from a substituted vinyl compound comprising at least one basic atom, such as more particularly dialkylaminoalkyl methacrylate and acrylate or dialkylaminoalkylmethacrylamide and -acrylamide. Such compounds are described in United States Patent 3 836 537.
(2) Polymers including units deriving:
   a) from at least one monomer chosen from acrylamides or methacrylamides substituted on the nitrogen atom by an alkyl group,
   b) from at least one acidic comonomer containing one or more reactive carboxylic groups, and
   c) from at least one basic comonomer, such as esters comprising primary, secondary, tertiary and quaternary amine substituents of acrylic and methacrylic acids and the quaternization product of dimethylaminoethyl methacrylate with dimethyl or diethyl sulfate.

The more particularly preferred N-substituted acrylamides or methacrylamides according to the invention are the compounds in which the alkyl groups comprise from 2 to 12 carbon atoms and more particularly N-ethylacrylamide, N-(*tert*-butyl)acrylamide, N-(*tert*-octyl)acrylamide, N-octylacrylamide, N-decylacrylamide, N-dodecylacrylamide and the corresponding methacrylamides.

The acidic comonomers are chosen more particularly from acrylic, methacrylic, crotonic, itaconic, maleic or fumaric acid and also the alkyl monoesters, the alkyl group having from 1 to 4 carbon atoms, of maleic or fumaric acid or anhydride.

The preferred basic comonomers are aminoethyl, butylaminoethyl, N,N'-dimethylaminoethyl and N-(*tert*-butyl)aminoethyl methacrylates.

Use is made in particular of the copolymers for which the INCI name is Octylacrylamide/acrylates/butylaminoethyl methacrylate copolymer, such as the products sold under the names Amphomer^{®}, Amphomer^{®} LV71 or Balance^{®} 47 by AkzoNobel.

(3) Partially or completely acylated and crosslinked polyaminoamides deriving from polyaminoamides of general formula: 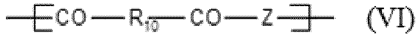 in which R₁₀ represents a divalent group derived from a saturated dicarboxylic acid, from an aliphatic mono- or dicarboxylic acid comprising an ethylenic double bond, from an ester of a lower alkanol having from 1 to 6 carbon atoms of these acids, or from a group deriving from the addition of any one of the said acids to a bis-primary or bis-secondary amine, and Z denotes a group deriving from a bis-primary, mono- or bis-secondary polyalkylenepolyamine and preferably represents:
a) in the proportions of from 60 mol% to 100 mol%, the group 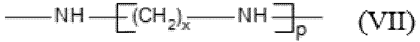
   where x = 2 and p = 2 or 3, or else x = 3 and p = 2,
   this group deriving from diethylenetriamine, triethylenetetramine or dipropylenetriamine;
b) in the proportions of from 0 mol% to 40 mol%, the group (VII) above in which x = 2 and p = 1 and which derives from ethylenediamine, or the group deriving from piperazine: 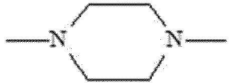
c) in the proportions of from 0 mol% to 20 mol%, the group -NH-(CH₂)₆-NH-deriving from hexamethylenediamine,
these polyaminoamides being crosslinked by addition reaction of a bifunctional crosslinking agent chosen from epihalohydrins, diepoxides, dianhydrides or bisunsaturated derivatives, by means of from 0.025 to 0.35 mol of crosslinking agent per amine group of the polyaminoamide, and acylated by reaction with acrylic acid, chloroacetic acid or an alkane sultone or their salts.

The saturated carboxylic acids are preferably chosen from acids having from 6 to 10 carbon atoms, such as adipic acid, 2,2,4-trimethyladipic acid and 2,4,4-trimethyladipic acid, terephthalic acid or acids comprising an ethylenic double bond, such as, for example, acrylic acid, methacrylic acid or itaconic acid.

The alkane sultones used in the acylation are preferably propane or butane sultone and the salts of the acylating agents are preferably the sodium or potassium salts.

(4) Polymers comprising zwitterionic units of formula: 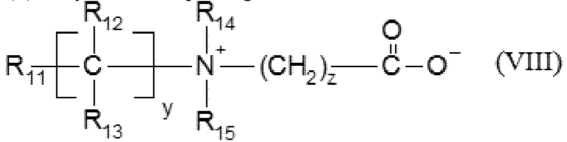 in which R₁₁ denotes a polymerizable unsaturated group, such as an acrylate, methacrylate, acrylamide or methacrylamide group, y and z represent an integer from 1 to 3, R₁₂ and R₁₃ represent a hydrogen atom or a methyl, ethyl or propyl group, and R₁₄ and R₁₅ represent a hydrogen atom or an alkyl group such that the sum of the carbon atoms in R₁₄ and R₁₅ does not exceed 10.
The polymers comprising such units can also comprise units derived from non-zwitterionic monomers, such as dimethyl- or diethylaminoethyl acrylate or methacrylate or alkyl acrylates or methacrylates, acrylamides or methacrylamides, or vinyl acetate.
Mention may be made, by way of example, of methyl methacrylate/methyl dimethylcarboxymethylammonioethyl methacrylate copolymers, such as the product sold under the name Diaformer Z-301N or Z-301W by Clariant (INCI name: Acrylates copolymer).
5) Polymers derived from chitosan comprising monomer units corresponding to the following formulae: 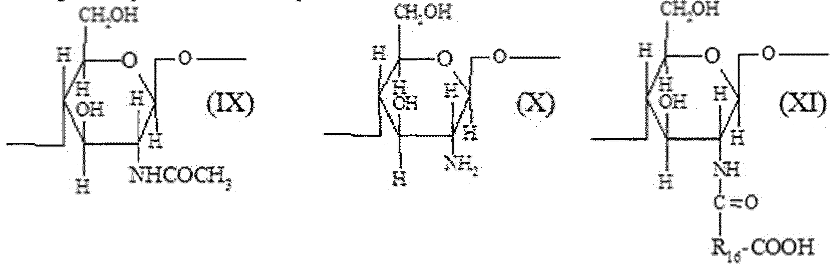 the unit (IX) being present in proportions of between 0% and 30%, the unit (X) in proportions of between 5% and 50% and the unit (XI) in proportions of between 30% and 90%, it being understood that, in this unit (XI), R₁₆ represents a group of formula: 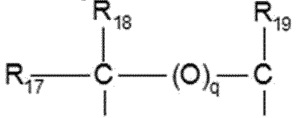
   in which, if q = 0, R₁₇, R₁₈ and R₁₉, which are identical or different, each represent a hydrogen atom, a methyl, hydroxyl, acetoxy or amino residue, a monoalkylamine residue or a dialkylamine residue, which are optionally interrupted by one or more nitrogen atoms and/or optionally substituted by one or more amine, hydroxyl, carboxyl, alkylthio or sulfonic groups, or an alkylthio residue in which the alkyl group carries an amino residue, at least one of the R₁₇, R₁₈ and R₁₉ groups being, in this case, a hydrogen atom;
   or, if q = 1, R₁₇, R₁₈ and R₁₉ each represent a hydrogen atom, and also the salts formed by these compounds with bases or acids.
(6) Polymers containing units corresponding to the general formula (XII) are, for example, described in French Patent 1 400 366: 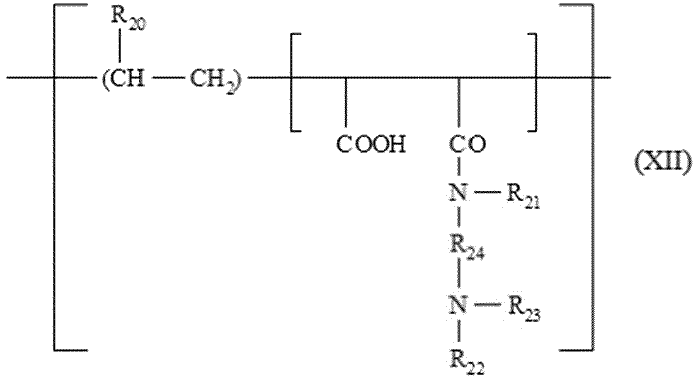 in which R₂₀ represents a hydrogen atom or a CH₃O, CH₃CH₂O or phenyl group, R₂₁ denotes a hydrogen atom or a lower alkyl group, such as methyl or ethyl, R₂₂ denotes a hydrogen atom or a lower C₁-C₆ alkyl group, such as methyl or ethyl, and R₂₃ denotes a lower C₁-C₆ alkyl group, such as methyl or ethyl, or a group corresponding to the formula: -R₂₄-N(R₂₂)₂, R₂₄ representing a -CH₂-CH₂-, -CH₂-CH₂-CH₂- or -CH₂-CH(CH₃)- group and R₂₂ having the meanings mentioned above.
(7) Polymers derived from the N-carboxyalkylation of chitosan, such as N- (carboxymethyl)chitosan or N-(carboxybutyl)chitosan, such as, for example, the product sold under the name Chitoglycan by Sinerga SpA (INCI name: Carboxymethyl chitosan).
(8) Amphoteric polymers of the -D-X-D-X- type chosen from:
   a) polymers obtained by reaction of chloroacetic acid or sodium chloroacetate with compounds comprising at least one unit of formula: -D-X-D-X-D-
      (XIII)
      where D denotes a group 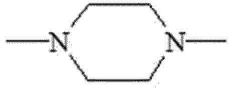
      and X denotes the symbol E or E', E or E', which are identical or different, denoting a divalent group which is a straight- or branched-chain alkylene group comprising up to 7 carbon atoms in the main chain which is unsubstituted or substituted by hydroxyl groups and which can additionally comprise oxygen, nitrogen and sulfur atoms and from 1 to 3 aromatic and/or heterocyclic rings; the oxygen, nitrogen and sulfur atoms being present in the form of ether, thioether, sulfoxide, sulfone, sulfonium, alkylamine or alkenylamine groups or hydroxyl, benzylamine, amine oxide, quaternary ammonium, amide, imide, alcohol, ester and/or urethane groups,
   b) polymers of formula:

      -D-X-D-X- (XIII')

      where D denotes a group 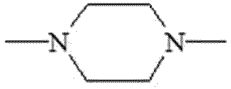
      and X denotes the symbol E or E' and at least once E'; E having the meaning indicated above and E' being a divalent group which is a straight- or branched-chain alkylene group having up to 7 carbon atoms in the main chain which is unsubstituted or substituted by one or more hydroxyl groups and which comprises one or more nitrogen atoms, the nitrogen atom being substituted by an alkyl chain optionally interrupted by an oxygen atom and necessarily comprising one or more carboxyl functional groups or one or more hydroxyl functional groups and betainized by reaction with chloroacetic acid or sodium chloroacetate.
(9) (C₁-C₅)Alkyl vinyl ether/maleic anhydride copolymers partially modified by semiamidation with an N,N-dialkylaminoalkylamine, such as N,N-dimethylaminopropylamine, or by semiesterification with an N,N-dialkylaminoalkanol. These copolymers can also comprise other vinyl comonomers, such as vinylcaprolactam.

Mention will be made, among the amphoteric fixing polymers mentioned above which are the most particularly preferred according to the invention, of those of family (3), such as the copolymers for which the INCI name is Octylacrylamide/acrylates/butylaminoethyl methacrylate copolymer, such as the products sold under the name Amphomer^{®}, Amphomer^{®} LV 71 or Balance^{®} 47 by AkzoNobel, and those of family (4), such as methyl methacrylate/methyl dimethylcarboxymethylammonioethyl methacrylate copolymers, sold, for example, under the name Diaformer Z-301N or Z-301W by Clariant.

Preferably, the fixing polymer(s) are chosen from non-ionic fixing polymers and their mixtures, and more preferentially from:
- polyalkyloxazolines;
- vinyl acetate homopolymers;
- vinyl acetate copolymers, such as, for example, copolymers of vinyl acetate and of acrylic ester, copolymers of vinyl acetate and of ethylene, or copolymers of vinyl acetate and of maleic ester, for example of dibutyl maleate;
- acrylic ester homopolymers and copolymers, such as, for example, copolymers of alkyl acrylates and of alkyl methacrylates, such as the products provided by Röhm GmbH under the name Eudragit^{®} NE 30 D (INCI name: Acrylates copolymer);
- copolymers of acrylonitrile and of a non-ionic monomer chosen, for example, from butadiene and alkyl (meth)acrylates;
- styrene homopolymers;
- styrene copolymers, such as, for example, copolymers of styrene, of alkyl methacrylate and of alkyl acrylate; copolymers of styrene and of butadiene; or copolymers of styrene, of butadiene and of vinylpyridine;
- polyamides;
- vinyllactam homopolymers, such as vinylpyrrolidone homopolymers, for example sold under the names Luviskol^{®} K30 powder by BASF or PVP K30L or K60 solution or K90 by ISP, or such as the polyvinylcaprolactam sold under the name Luviskol^{®} Plus by BASF (INCI name: PVP);
- vinyllactam copolymers, such as a poly(vinylpyrrolidone/vinyllactam) copolymer sold under the trade name Luvitec^{®} VPC 55K65W by BASF, poly(vinylpyrrolidone/vinyl acetate) copolymers, such as those sold under the names PVP/VA^{®} S630L, E735, E635 and W735 by ISP and Luviskol^{®} VA 73, VA 64 and VA 37 by BASF (INCI name: VP/VA copolymer), and vinylpyrrolidone/methacrylamide/vinylimidazole terpolymers, such as, for example, that sold under the name Luviset^{®} Clear by BASF (INCI name: VP/methacrylamide/vinyl imidazole copolymer);
- and their mixtures.

The alkyl groups of the non-ionic polymers mentioned above preferably have from 1 to 6 carbon atoms.

Use may also be made, according to the invention, of fixing polymers of grafted silicone type comprising a polysiloxane portion and a portion constituted of a non-silicone organic chain, one of the two portions constituting the main chain of the polymer and the other being grafted to said main chain.

These polymers are described, for example, in Patent Applications EP-A-0 412 704, EP-A-0 412 707, EP-A-0 640 105 and WO 95/00578, EP-A-0 582 152 and WO 93/23009, and Patents US 4 693 935, US 4 728 571 and US 4 972 037.

These polymers can be amphoteric, anionic or non-ionic and they are preferably anionic or non-ionic.

Such polymers are, for example, copolymers capable of being obtained by radical polymerization starting from the monomer mixture formed of:
a) from 50% to 90% by weight of *tert*-butyl acrylate,
b) from 0% to 40% by weight of acrylic acid,
c) from 5% to 40% by weight of a silicone macromer of formula: 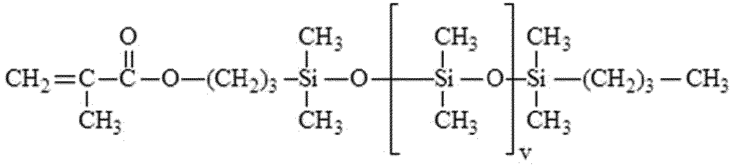 where v is a number ranging from 5 to 700, the percentages by weight being calculated with respect to the total weight of the monomers.

Other examples of grafted silicone polymers are in particular polydimethylsiloxanes (PDMSs) to which mixed polymer units of the poly((meth)acrylic acid) type and of the poly(alkyl (meth)acrylate) type are grafted via a thiopropylene-type connecting link and polydimethylsiloxanes (PDMSs) to which polymer units of the poly(isobutyl (meth)acrylate) type are grafted via a thiopropylene-type connecting link.

Grafted silicone polymers are, for example, sold under the names Silicone Plus Polymer^{®} VS80 and VA70 by 3M (INCI names: Polysilicone-8 and Polysilicone-7 respectively).

Mention may be made, as other type of silicone fixing polymer, of the product Luviflex^{®} Silk sold by BASF (INCI name: PEG/PPG-25/25 dimethicone/acrylates copolymer).

Use may also be made, as fixing polymers, of functionalized or non-functionalized and silicone or non-silicone cationic, non-ionic, anionic or amphoteric polyurethanes, or their mixtures.

The polyurethanes particularly targeted by the present invention are those described in Patent Applications EP 0 751 162, EP 0 637 600, EP 0 648 485 and FR 2 743 297, of which the Applicant Company is the proprietor, and also in Patent Applications EP 0 656 021 and WO 94/03510 of BASF and EP 0 619 111 of National Starch.

Mention may be made, as polyurethanes particularly highly suitable in the present invention, of the products sold under the names Luviset^{®} PUR and Luviset^{®} Si PUR by BASF (INCI names: Polyurethane-1 and Polyurethane-6 respectively).

Preferably, the fixing polymer(s) are chosen from non-ionic fixing polymers and their mixtures, more preferentially from vinyllactam homopolymers, such as vinylpyrrolidone homopolymers or polyvinylcaprolactam, and vinyllactam copolymers, such as a poly(vinylpyrrolidone/vinyllactam) copolymer, poly(vinylpyrrolidone/vinyl acetate) copolymers or poly(vinylpyrrolidone/vinyl acetate/vinyl propionate) terpolymers, and their mixtures.

Advantageously, the fixing polymer(s) are chosen from vinyllactam homopolymers, such as vinylpyrrolidone homopolymers and polyvinylcaprolactam, and their mixtures, and, better still, the fixing polymer is polyvinylcaprolactam.

The total amount of the fixing polymer(s), present in the cosmetic composition contained in the aerosol device according to the invention, preferably ranges from 0.1% to 15% by weight, more preferentially from 0.5% to 10% by weight and better still from 1% to 8% by weight, with respect to the total weight of the composition.

### The powders

The cosmetic composition contained in the aerosol device according to the present invention additionally comprises one or more powders.

The powders that can be used according to the present invention are preferably water-insoluble.

Within the meaning of the present invention, the term "water-insoluble" is understood to mean a compound, the solubility of which at spontaneous pH in water at 25°C and at atmospheric pressure is less than 0.1%.

The powders can be chosen from fillers, pigments and their mixtures.

Preferably, the composition according to the invention contains one or more styling powder(s).

Within the meaning of the present invention, the term "styling powder" is understood to mean a powder exhibiting a capacity for shaping the head of hair or for the durability of this shaping.

The capacity of the powder for shaping or for the durability of the shaping may in particular be due to its chemical nature and/or its geometric shape and/or its arrangement configuration during the deposition on the keratinous fibre. This is because the irregularities created at the surface of the hairs promote the inter-attachment of the fibres.

The powder can be of any shape, such as lamellar, spherical or oblong, whatever the crystallographic form (for example cubic, hexagonal, orthorhombic, rhombohedral or tetragonal). In a preferred embodiment, the powders are not spherical.

The number-average size of the powder can vary from 0.001 to 50 µm, better still from 0.002 to 40 µm and even more preferentially from 0.003 to 35 µm.

This number-average size corresponds to the size measured from the statistical distribution of the particle sizes for half of the total number of the particles. This size is denoted D50.

In addition, the number-average size of these particles can be measured in the form of a mean value by an observation method with an optical microscope, with an electron microscope, or a particle size analyser using laser diffraction.

In the case where the particles do not exhibit a spherical shape, their number-average size can be determined in the form of a mean of the longest or shortest diameter or of the thickness.

The powder(s) according to the invention can be mineral or organic, preferably mineral.

The powder is preferably chosen from pigments, fillers and their mixtures.

According to a first specific embodiment of the invention, the cosmetic composition contained in the aerosol device according to the present invention comprises one or more powders, at least one of which is chosen from fillers and their mixtures. In this embodiment, the filler is preferably a styling powder.

According to a second specific embodiment of the invention, the cosmetic composition contained in the aerosol device according to the present invention comprises one or more powders, at least one of which is chosen from pigments and their mixtures. In other words, according to this embodiment, the cosmetic composition comprises at least one pigment.

According to a third embodiment of the invention, the cosmetic composition contained in the aerosol device according to the present invention comprises one or more pigments and one or more fillers. In this embodiment, the filler is preferably a styling powder.

Within the meaning of the present invention, the term "filler" is understood to mean natural or synthetic and white or colourless particles whichs exhibit any shape and which are insoluble in the medium of the composition, whatever the temperature at which the composition is manufactured.

The fillers can be organic or inorganic, and of any shape, such as lamellar, spherical or oblong, whatever the crystallographic form (for example cubic, hexagonal, orthorhombic, rhombohedral or tetragonal). In a preferred embodiment, the fillers are not spherical.

Within the meaning of the present invention, the term "pigment" is understood to mean organic or mineral and white or coloured particles of any shape which are insoluble in physiological medium and which confer a colouring on the composition.

The term "mineral" encompasses natural or synthetic chemical compounds which are inorganic. Mineral substances exist mainly in a crystalline form.

Mention may in particular be made, as examples of mineral or inorganic powder, of:
- fillers, such as metal carbonates, oxides and sulfates, for example those of alkaline earth metals, aluminium, gallium and indium; silicates; modified or unmodified silicas; sericite, synthetic fluorphlogopite, talc; natural or synthetic mica, in particular white mica, gold mica, red mica, black mica or lithium oxide-coated mica; calcium phosphate, silicic acid, silicic anhydride, silicon carbide, metal salts of tungstic acid, magnesium aluminate, bentonite, zeolites, smectite, hydroxyapatite, ceramic powder, boron nitride and glass or ceramic microcapsules;
- specific composite fillers, such as those sold under the names Excel Mica, Excel Pearl and the powder La Vie by Miyoshi Kasei Inc.;
- white pigments, such as titanium dioxide, zinc oxide, zirconium oxide and cerium oxide;
- coloured pigments, such as red iron oxide, yellow iron oxide, black iron oxide, chromium oxide, chromium hydroxide, Prussian blue, ultramarine blue, chromium hydrate, ferric blue, inorganic blue pigments, carbon black, lower titanium oxides, manganese violet, cobalt violet and metal powders, such as aluminium powder and copper powder;
- pearlescent pigments, such as bismuth oxychloride, titanium oxide-coated mica, pearl essence, the powder prepared by coating synthetic mica with titanium dioxide, the powder prepared by coating silica flakes with titanium dioxide, which is available under the trade name Metashine from Nippon Sheet Glass Co. Ltd, the powder prepared by coating alumina flakes with tin oxide and titanium dioxide, the powder prepared by coating aluminium flakes with titanium dioxide, the powder prepared by coating copper flakes with silica, sold by Eckert Inc. USA, the powder prepared by coating bronze flakes with silica, and the powder prepared by coating aluminium flakes with silica;
- ultrafine powder, exhibiting a mean particle size of less than 0.1 µm, such as ultrafine titanium dioxide, ultrafine zinc oxide, ultrafine iron oxide and ultrafine cerium oxide;
- other powders, such as the luminescent powder sold under the trade name Luminova Series by Mitsui & Co. Ltd., aluminium powder, stainless steel powder, tourmaline powder and amber powder; and
- one of their mixtures.

Examples of organic powders are wool powder, polyamide powder (Nylon^{®} or Orgasol^{®} from Arkema), polyester powder, polyethylene powder, polypropylene powder, polystyrene powder, polyurethane powder, benzoguanamine powder, polymethyl benzoguanamine powder, tetrafluoroethylene powder, poly(methyl methacrylate) powder, cellulose powder, silk powder, silicone powder, silicone rubber powder, powders of synthetic resins, such as a styrene/acrylate copolymer, a divinylbenzene/styrene copolymer, a vinyl resin, a urea resin, a phenolic resin, a fluororesin, tetrafluoroethylene (Teflon^{®}) polymers, a silicone resin, an acrylic resin, a melamine resin, an epoxy resin and a polycarbonate resin, polymeric hollow microspheres, such as those of poly(vinylidene chloride)/acrylonitrile, for example Expancel^{®} (Nobel Industrie), or acrylic acid copolymers (Polytrap^{®} from Dow Corning), silicone resin microbeads (for example Tospearls^{®} from Toshiba), particles formed of polyorganosiloxane elastomers, microcrystalline fibre powder, starch powder, acylated lysine powder, poly-β-alanine, lauryllysine, long-chain alkyl phosphate metal salt powder, metal soap powder, Colour Index (CI) yellow pigments, CI orange pigments and tar-derived pigments prepared in the lake form, and pigments existing in the natural state prepared in the lake form.

The doped or undoped composite powder may also be suitable as base powder intended to undergo a surface treatment. Examples of the latter encompass the powder prepared by coating inorganic dye pigments, such as red iron oxide, with silicic anhydride, powders prepared by coating Nylon with white pigments and powders prepared by coating fillers with ultrafine white pigments.

The mineral powder(s) according to the invention can optionally be surface-modified with organic compounds.

More preferentially, the powder is a mineral powder constituted of one or more water-insoluble mineral compounds.

The water-insoluble mineral compound(s) are preferably chosen from metal carbonates, oxides and sulfates, silicates, modified or unmodified silicas, mica, talc and their mixtures.

Mention may more particularly be made, by way of examples, of the carbonates, oxides and sulfates of alkaline earth metals, such as beryllium, magnesium, calcium, strontium, barium and radium, better still magnesium and calcium; the oxides, sulfates and carbonates of aluminium, gallium and indium; silicates, such as kaolinite or kaolins (natural silicates containing kaolinite), silicates containing magnesium, in particular those containing an amount of magnesium of greater than 10% by weight (on a dry basis), expressed as magnesium oxide, such as Li-Mg-Na silicates, for example Laponite XLG provided by Rockwood; modified or unmodified silicas, better still modified silicas; mica; talc; and their mixtures.

Among the modified silicas, it is preferred to use surface-treated silicas, such as hydrophobic silicas, such as, for example, hydrophobic fumed silica of nanometric size and surface-treated with hexamethyldisilazane, such as the silica sold under the trade name Aerosil R812S or Aerosil R972 by Evonik or HDK H115 by Wacker, or hydrophobic fumed silica surface-treated with dimethylsilane.

More preferentially, the powder(s) are chosen from fillers and more preferentially from calcium carbonate, magnesium carbonate, alumina, barium sulfate, magnesium oxide, kaolinite or kaolins, modified or unmodified silicas, better still modified silicas and even better still hydrophobic fumed silica of nanometric size and surface-treated with hexamethyldisilazane or hydrophobic fumed silica surface-treated with dimethylsilane; mica; talc; and their mixtures.

According to a preferred embodiment, the cosmetic composition contained in the aerosol device according to the present invention comprises calcium carbonate.

According to yet another specific embodiment, the cosmetic composition contained in the aerosol device according to the present invention comprises calcium carbonate and one or more pigments.

The total amount of the powder(s), present in the cosmetic composition contained in the aerosol device according to the invention, preferably ranges from 0.1% to 15% by weight, more preferentially from 0.5% to 10% by weight and better still from 1% to 8% by weight, with respect to the total weight of the composition.

The ratio by weight of the total amount of the powder(s) to the total amount of the fixing polymer(s), which are present in the cosmetic composition contained in the aerosol device according to the invention, is greater than or equal to 1 and preferably ranges from 1 to 5.

### The silicones

The cosmetic composition contained in the aerosol device according to the present invention additionally comprises at least 0.05% by weight, with respect to the total weight of the composition, of one or more polydimethylsiloxanes comprising dimethylsilanol end groups.

Mention may be made, as examples of polydimethylsiloxanes comprising dimethylsilanol end groups, also known as polydimethylsiloxanes comprising α,ω-silanol groups, also known under the name of dimethiconol (CTFA), of the oils of the 48 series from Rhodia.

Mention may be made of dimethiconol emulsions, for example with anionic surfactants, preferably having a small particle size, such as less than 500 nm, preferably less than 200 nm.

Products which can more particularly be used in accordance with the invention are mixtures, such as the mixtures formed from a polydimethylsiloxane comprising dimethylsilanol end groups, or dimethiconol (CTFA), and from a cyclic polydimethylsiloxane, also known as cyclomethicone (CTFA), or linear polydimethylsiloxane, also known as dimethicone (CTFA), such as the product Q2 1401 or also the product 1503 Fluid sold by Dow Corning.

The total amount of the polydimethylsiloxane(s) comprising dimethylsilanol end groups present in the cosmetic composition contained in the aerosol device according to the invention is preferably greater than or equal to 0.1% by weight and more preferentially ranges from 0.1% to 1% by weight, with respect to the total weight of the composition.

The ratio by weight of the total amount of the fixing polymer(s) to the total amount of the polydimethylsiloxane(s) comprising dimethylsilanol end groups which are present in the cosmetic composition contained in the aerosol device according to the invention is preferably greater than or equal to 1, more preferentially greater than or equal to 5, better still ranges from 5 to 25 and more preferentially still from 10 to 20.

The cosmetic composition contained in the aerosol device according to the present invention can optionally also comprise one or more additional silicones other than the polydimethylsiloxanes comprising dimethylsilanol end groups of the invention.

Preferably, the additional silicone(s) are chosen from polydialkylsiloxanes, in particular polydimethylsiloxanes (PDMS) other than the polydimethylsiloxanes comprising dimethylsilanol end groups of the invention, and organomodified polysiloxanes comprising at least one functional group chosen from amino groups, oxyalkylene groups, aryl groups and alkoxy groups.

Organopolysiloxanes are defined in greater detail in the work by Walter Noll, Chemistry and Technology of Silicones (1968), Academic Press. They can be volatile or non-volatile.

The non-volatile silicones which can be used in the composition according to the invention can preferably be non-volatile polydialkylsiloxanes, polyorganosiloxanes modified by organofunctional groups chosen from amine groups, aryl groups, oxyalkylene groups and alkoxy groups, and also their mixtures.

These silicones are more particularly chosen from polydialkylsiloxanes, among which mention may be made mainly of polydimethylsiloxanes comprising trimethylsilyl end groups. The viscosity of the silicones is measured at 25°C according to Standard ASTM 445, Appendix C.

Mention may be made, among these polydialkylsiloxanes, in a non-limiting way, of the following commercial products:
- the Silbione^{®} oils of the 47 and 70 047 series or the Mirasil^{®} oils sold by Rhodia;
- the oils of the Mirasil^{®} series sold by Rhodia;
- the oils of the 200 series from Dow Corning;
- the Viscasil^{®} oils from General Electric and certain oils of the SF series (SF 96, SF 18) from General Electric.

The organomodified silicones which can be used in accordance with the invention are silicones as defined above and comprising, in their structure, one or more organofunctional groups attached via a hydrocarbon group.

The organomodified silicones can be polydiarylsiloxanes, in particular polydiphenylsiloxanes, polydialkylsiloxanes and polyalkylarylsiloxanes functionalized by the abovementioned organofunctional groups.

The polyalkylarylsiloxanes are particularly chosen from linear and/or branched polydimethyl/methylphenylsiloxanes and polydimethyl/diphenylsiloxanes with a viscosity ranging from 1.10⁻⁵ to 5.10⁻² m²/s at 25°C.

Mention may be made, among these polyalkylarylsiloxanes, by way of examples, of the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Mention may also be made, among the organomodified silicones, of the polyorganosiloxanes comprising:
- substituted or unsubstituted amino groups, such as the products sold under the names GP 4 Silicone Fluid and GP 7100 by Genesee. The substituted amino groups are in particular C₁-C₄ aminoalkyl groups;
- alkoxylated groups, such as the product sold under the names Silicone Copolymer F-755 by SWS Silicones and Abil Wax^{®} 2428, 2434 and 2440 by Goldschmidt;
- oxyalkylene groups, in particular oxyethylene groups, such as the product sold under the name Xiameter OFX-0193 Fluid by Dow Corning.

The volatile silicones are more particularly chosen from those with a boiling point of between 60°C and 260°C, and even more particularly from:
- cyclic silicones comprising from 3 to 7 silicon atoms and preferably from 4 to 6.

They are, for example, octamethylcyclotetrasiloxane, sold in particular under the name Volatile Silicone 7207 by Union Carbide or Silbione 70045 V 2 by Rhodia, decamethylcyclopentasiloxane, sold under the name Volatile Silicone 7158 by Union Carbide or Silbione 70045 V 5 by Rhodia, and their mixtures.

Mention may also be made of cyclocopolymers of the dimethylsiloxane/methylalkylsiloxane type, such as Volatile Silicone FZ 3109 sold by Union Carbide, of chemical structure: 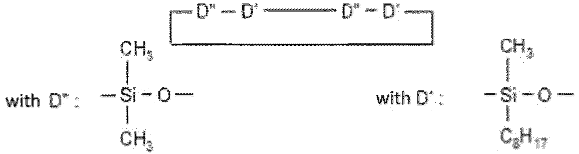 Mention may also be made of mixtures of cyclic silicones with organic compounds derived from silicon, such as the mixture of octamethylcyclotetrasiloxane and tetratrimethylsilylpentaerythritol (50/50) and the mixture of octamethylcyclotetrasiloxane and oxy-1,1'-bis[2,2,2',2',3,3'-hexa(trimethylsilyloxy)neopentane].
- volatile linear silicones having from 2 to 9 silicon atoms and possessing a viscosity of less than or equal to 5.10⁻⁶ m²/s at 25°C. These are, for example, hexamethyldisiloxane, octamethyltrisiloxane and decamethyltetrasiloxane, sold in particular under the name SH 200 by Toray Silicone. Silicones coming within this category are also described in the paper published in Cosmetics and Toiletries, Vol. 91, Jan. 76, pp. 27-32, Todd & Byers, "Volatile Silicone Fluids for Cosmetics". Preferably, the volatile linear silicones contain from 2 to 7 silicon atoms and better still from 3 to 6 silicon atoms.

Preferably, the additional volatile silicones are chosen from cyclic silicones comprising from 4 to 6 silicon atoms and linear silicones containing from 4 to 6 silicon atoms.

Preferably, the additional non-volatile silicones are chosen from polydialkylsiloxanes, more particularly polydimethylsiloxanes other than the polydimethylsiloxanes comprising dimethylsilanol end groups of the invention, which are or are not organomodified.

The additional silicone(s) other than the polydimethylsiloxanes comprising dimethylsilanol end groups of the invention which can be used in the cosmetic composition according to the invention can be chosen from aminosilicones.

The term "aminosilicone" denotes any silicone comprising at least one primary, secondary or tertiary amine or a quaternary ammonium group.

The weight-average molecular weights of these aminosilicones can be measured by gel permeation chromatography (GPC) at ambient temperature (25°C) as polystyrene equivalent. The columns used are µ styragel columns. The eluent is THF and the flow rate is 1 ml/min. 200 µl of a 0.5% by weight solution of silicone in THF are injected. Detection is carried out by refractometry and UV spectrometry.

Preferably, the aminosilicone(s) capable of being employed in the context of the invention are chosen from:
a) the polysiloxanes corresponding to the formula (A): 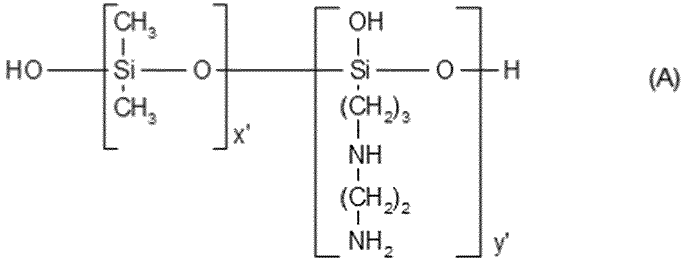 in which x' and y' are integers such that the weight-average molecular weight (Mw) is of between 5000 and 500 000 approximately;
b) the aminosilicones corresponding to formula (B):

   R'ₐG₃₋ₐ-Si(OSiG₂)ₙ-(OSiG_{b}R'_{2-b})ₘ-O-SiG₃₋ₐ-R'ₐ (B)

   in which:
   - G, which is identical or different, denotes a hydrogen atom or a phenyl, OH, C₁-C₈ alkyl, for example methyl, or C₁-C₈ alkoxy, for example methoxy, group,
   - a, which is identical or different, denotes 0 or an integer from 1 to 3, in particular 0,
   - b denotes 0 or 1, in particular 1,
   - m and n are numbers such that the sum (n + m) varies from 1 to 2000 and especially from 50 to 150, it being possible for n to denote a number from 0 to 1999 and in particular from 49 to 149 and it being possible for m to denote a number from 1 to 2000 and in particular from 1 to 10;
   - R', which is identical or different, denotes a monovalent radical of formula -C_{q}H_{2q}L in which q is a number ranging from 2 to 8 and L is an optionally quaternized amino group chosen from the following groups:

      -N(R")₂; -N⁺(R")₃ A⁻; -NR"-Q-N(R")₂

      and

      -NR"-Q-N⁺(R")₃ A⁻,

      in which R", which is identical or different, denotes hydrogen, phenyl, benzyl or a saturated monovalent hydrocarbon radical, for example a C₁-C₂₀ alkyl radical; Q denotes a linear or branched group of formula CᵣH₂ᵣ, r being an integer ranging from 2 to 6, preferably from 2 to 4; and A⁻ represents a cosmetically acceptable anion, in particular a halide anion, such as a fluoride, chloride, bromide or iodide anion.

Preferably, the aminosilicones are chosen from the aminosilicones of formula (B). Preferably, the aminosilicones of formula (B) are chosen from the aminosilicones corresponding to the following formulae (C), (D), (E), (F) and/or (G).

According to a first embodiment, the aminosilicones corresponding to the formula (B) are chosen from the silicones known as "trimethylsilyl amodimethicone" corresponding to the formula (C): 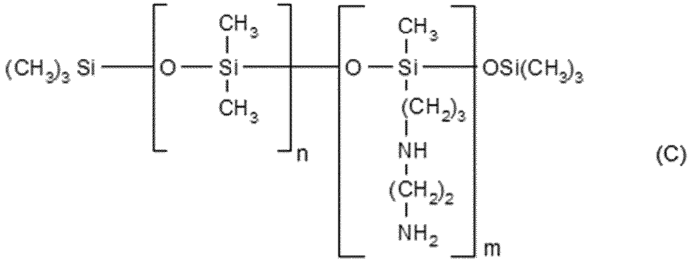 in which m and n are numbers such that the sum (n + m) varies from 1 to 2000 and especially from 50 to 150, it being possible for n to denote a number from 0 to 1999 and in particular from 49 to 149 and it being possible for m to denote a number from 1 to 2000 and in particular from 1 to 10.

According to a second embodiment, the aminosilicones corresponding to the formula (B) are chosen from the silicones of following formula (D): 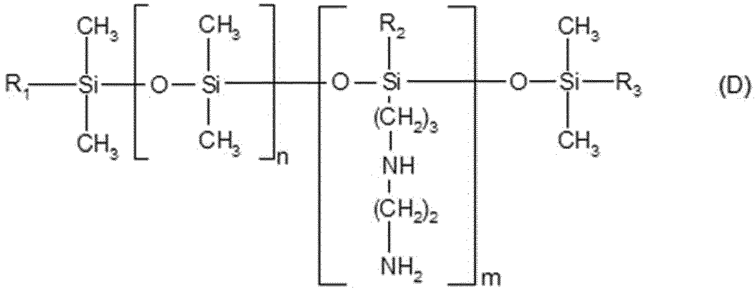 in which:
- m and n are numbers such that the sum (n + m) varies from 1 to 1000, especially from 50 to 250 and more particularly from 100 to 200, it being possible for n to denote a number from 0 to 999, in particular from 49 to 249 and more particularly from 125 to 175 and it being possible for m to denote a number from 1 to 1000, in particular from 1 to 10 and more particularly from 1 to 5;
- R₁, R₂ and R₃, which are identical or different, represent a hydroxyl or C₁-C₄ alkoxy radical, at least one of the radicals R₁ to R₃ denoting an alkoxy radical.

Preferably, the alkoxy radical is a methoxy radical.

The hydroxy/alkoxy molar ratio preferably ranges from 0.2:1 to 0.4:1 and preferably from 0.25:1 to 0.35:1 and more particularly is equal to 0.3:1.

The weight-average molecular weight (Mw) of these silicones preferably ranges from 2000 to 1 000 000 and more particularly from 3500 to 200 000.

According to a third embodiment, the aminosilicones corresponding to the formula (B) are chosen from the silicones of following formula (E): 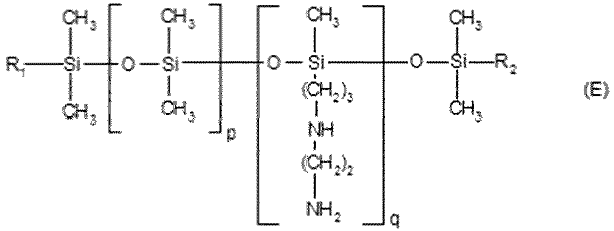 in which:
- p and q are numbers such that the sum (p + q) varies from 1 to 1000, in particular from 50 to 350 and more particularly from 150 to 250, it being possible for p to denote a number from 0 to 999, in particular from 49 to 349 and more particularly from 159 to 239 and it being possible for q to denote a number from 1 to 1000, in particular from 1 to 10 and more particularly from 1 to 5;
- R₁ and R₂, which are different, represent a hydroxyl or C₁-C₄ alkoxy radical, at least one of the radicals R₁ or R₂ denoting an alkoxy radical.

Preferably, the alkoxy radical is a methoxy radical.

The hydroxy/alkoxy molar ratio generally ranges from 1:0.8 to 1:1.1 and preferably from 1:0.9 to 1:1 and more particularly is equal to 1:0.95.

The weight-average molecular weight (Mw) of the silicone preferably ranges from 2000 to 200 000, more particularly still from 5000 to 100 000 and more particularly from 10 000 to 50 000.

The commercial products comprising silicones of structure (D) or (E) can include, in their composition, one or more other aminosilicones, the structure of which is different from the formula (D) or (E).

A product containing aminosilicones of structure (D) is provided by Wacker under the name Belsil^{®} ADM 652.

A product containing aminosilicones of structure (E) is provided by Wacker under the name Fluid WR 1300^{®}.

When these aminosilicones are employed, a particularly advantageous embodiment is their use in the form of an oil-in-water emulsion. The oil-in-water emulsion can comprise one or more surfactants. The surfactants can be of any nature but are preferably cationic and/or non-ionic. The number-average size of the silicone particles in the emulsion generally ranges from 3 nm to 500 nanometres. Preferably, in particular as aminosilicones of formula (E), use is made of microemulsions, the mean size of the particles of which ranges from 5 nm to 60 nanometres (limits included) and more particularly from 10 nm to 50 nanometres (limits included). Thus, use may be made according to the invention of the microemulsions of aminosilicone of formula (E) which are provided under the name Finish CT 96 E^{®} or SLM 28020^{®} by Wacker.

According to a fourth embodiment, the aminosilicones corresponding to the formula (B) are chosen from the silicones of following formula (F): 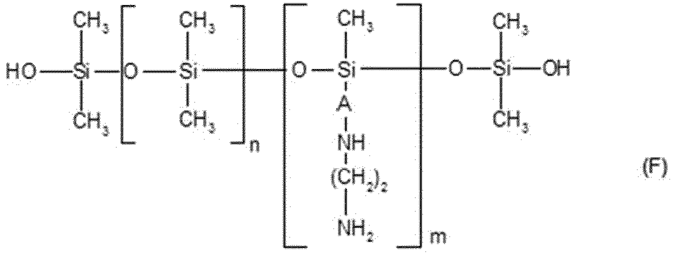 **in which:**
- m and n are numbers such that the sum (n + m) varies from 1 to 2000 and especially from 50 to 150, it being possible for n to denote a number from 0 to 1999 and in particular from 49 to 149 and it being possible for m to denote a number from 1 to 2000 and in particular from 1 to 10;
- A denotes a linear or branched alkylene radical having from 4 to 8 carbon atoms and preferably 4 carbon atoms. This radical is preferably linear.

The weight-average molecular weight (Mw) of these aminosilicones preferably ranges from 2000 to 1 000 000 and more particularly still from 3500 to 200 000.

A silicone corresponding to this formula is, for example, the Xiameter MEM 8299 Emulsion from Dow Corning.

According to a fifth embodiment, the aminosilicones corresponding to the formula (B) are chosen from the silicones of following formula (G): 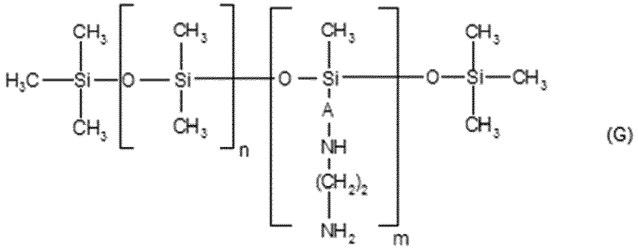 in which:
- m and n are numbers such that the sum (n + m) varies from 1 to 2000 and especially from 50 to 150, it being possible for n to denote a number from 0 to 1999 and in particular from 49 to 149 and it being possible for m to denote a number from 1 to 2000 and in particular from 1 to 10;
- A denotes a linear or branched alkylene radical having from 4 to 8 carbon atoms and preferably 4 carbon atoms. This radical is preferably branched.

The weight-average molecular weight (Mw) of these aminosilicones preferably ranges from 500 to 1 000 000 and more particularly still from 1000 to 200 000.

A silicone corresponding to this formula is, for example, DC2-8566 Amino Fluid from Dow Corning;
c) the aminosilicones corresponding to the formula (H): 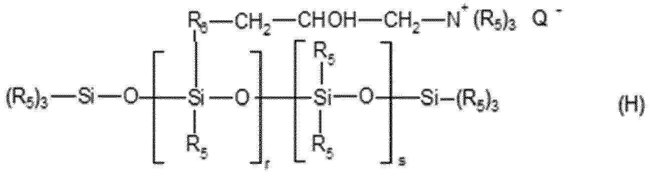 in which:
- R₅ represents a monovalent hydrocarbon radical having from 1 to 18 carbon atoms, and in particular a C₁-C₁₈ alkyl or C₂-C₁₈ alkenyl, for example methyl, radical;
- R₆ represents a divalent hydrocarbon radical, in particular a C₁-C₁₈ alkylene radical or a divalent C₁-C₁₈, for example C₁-C₈, alkyleneoxy radical connected to the Si via an SiC bond;
- Q⁻ is an anion, such as a halide ion, in particular a chloride ion, or an organic acid salt, in particular an acetate;
- r represents a mean statistical value ranging from 2 to 20 and in particular from 2 to 8;
- s represents a mean statistical value ranging from 20 to 200 and in particular from 20 to 50.

Such aminosilicones are described in particular in Patent US 4 185 087.
d) the silicones comprising a quaternary ammonium of following formula: 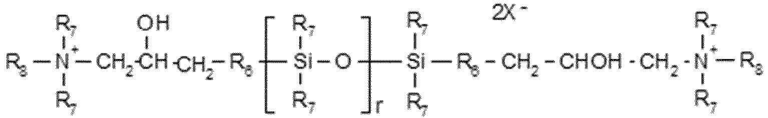 in which:
   - R₇, which are identical or different, represent a monovalent hydrocarbon radical having from 1 to 18 carbon atoms, and in particular a C₁-C₁₈ alkyl radical, a C₂-C₁₈ alkenyl radical or a ring comprising 5 or 6 carbon atoms, for example methyl;
   - R₆ represents a divalent hydrocarbon radical, in particular a C₁-C₁₈ alkylene radical or a divalent C₁-C₁₈, for example C₁-C₈, alkyleneoxy radical connected to the Si via an SiC bond;
   - R₈, which are identical or different, represent a hydrogen atom, a monovalent hydrocarbon radical having from 1 to 18 carbon atoms, and in particular a C₁-C₁₈ alkyl radical, a C₂-C₁₈ alkenyl radical or an -R₆-NHCOR₇ radical;
   - X⁻ is an anion, such as a halide ion, in particular a chloride ion, or an organic acid salt, in particular an acetate;
   - r represents a mean statistical value ranging from 2 to 200 and in particular from 5 to 100.
   These silicones are described, for example, in Application EP-A 0 530 974.
e) the aminosilicones of formula (J): 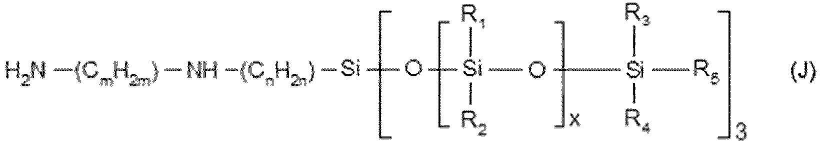 in which:
   - R₁, R₂, R₃ and R₄, which are identical or different, denote a C₁-C₄ alkyl radical or a phenyl group,
   - R₅ denotes a C₁-C₄ alkyl radical or a hydroxyl group,
   - n is an integer varying from 1 to 5,
   - m is an integer varying from 1 to 5, and
   - x is chosen in such a way that the amine number varies from 0.01 to 1 meq/g.
f) the multiblock polyoxyalkylenated aminosilicones of type (AB)ₙ, A being a polysiloxane block and B being a polyoxyalkylene block comprising at least one amine group.

The said silicones are preferably constituted of repeating units of following general formulae:

[-(SiMe₂O)ₓSiMe₂-R-N(R")-R'-O(C₂H₄O)ₐ(C₃H₆O)_{b}-R'-N(H)-R-]

or alternatively

[-(SiMe₂O)ₓSiMe₂-R-N(R")-R'-O(C₂H₄O)ₐ(C₃H₆O)_{b}-]

in which:
- a is an integer greater than or equal to 1, preferably ranging from 5 to 200 and more particularly ranging from 10 to 100;
- b is an integer of between 0 and 200, preferably ranging from 4 to 100 and more particularly between 5 and 30;
- x is an integer ranging from 1 to 10 000 and more particularly from 10 to 5000;
- R" is a hydrogen atom or a methyl;
- R, which are identical or different, represent a linear or branched divalent C₂-C₁₂ hydrocarbon radical, optionally comprising one or more heteroatoms, such as oxygen; preferably, R denotes an ethylene radical, a linear or branched propylene radical, a linear or branched butylene radical or a -CH₂CH₂CH₂OCH₂CH(OH)CH₂-radical; preferentially, R denotes a -CH₂CH₂CH₂OCH₂CH(OH)CH₂- radical;
- R', which are identical or different, represent a linear or branched divalent C₂-C₁₂ hydrocarbon radical, optionally comprising one or more heteroatoms, such as oxygen; preferably, R' denotes an ethylene radical, a linear or branched propylene radical, a linear or branched butylene radical or a -CH₂CH₂CH₂OCH₂CH(OH)CH₂-radical; preferentially, R' denotes -CH(CH₃)-CH₂-.

The siloxane blocks preferably represent from 50 mol% to 95 mol% of the total weight of the silicone, more particularly from 70 mol% to 85 mol%.

The amine content is preferably of between 0.02 and 0.5 meq/g of copolymer in a 30% solution in dipropylene glycol, more particularly between 0.05 and 0.2.

The weight-average molecular weight (Mw) of the silicone is preferably of between 5000 and 1 000 000 and more particularly between 10 000 and 200 000.

Mention may in particular be made of the silicones sold under the name Silsoft A-843 or Silsoft A+ by Momentive.
g) and their mixtures.

Preferably, the additional silicones other than the polydimethylsiloxanes comprising dimethylsilanol end groups of the invention are chosen from polydialkylsiloxanes, more particularly polydimethylsiloxanes, which are or are not organomodified, preferably non-organomodified polydimethylsiloxanes.

Preferably, the composition according to the invention comprises one or more additional silicone(s) other than the polydimethylsiloxanes comprising dimethylsilanol end groups of the invention.

When the cosmetic composition also comprises one or more additional silicones, the total amount of additional silicone other than the polydimethylsiloxanes comprising dimethylsilanol end groups of the invention, which are present in the said composition, preferably ranges from 0.1% to 10% by weight, more preferentially from 0.5% to 5% by weight and better still ranges from 0.8% to 2% by weight, with respect to the total weight of the composition.

When the cosmetic composition also comprises one or more additional silicones, the total amount of the silicones, which are present in the said composition (i.e. corresponding to the total amount of all of the silicones present in the cosmetic composition, that is to say polydimethylsiloxanes comprising dimethylsilanol end groups and the additional silicone(s)), is preferably greater than or equal to 0.5% by weight, more preferentially greater than or equal to 0.8% by weight and better still ranges from 0.8% to 3% by weight, with respect to the total weight of the composition.

### The organic solvents

The cosmetic composition contained in the aerosol device according to the present invention additionally comprises one or more organic solvents.

According to the invention, an organic solvent is liquid at ambient temperature (25°C) and atmospheric pressure (760 mmHg).

Preferably, the organic solvent(s) are chosen from linear or branched monoalcohols having from 1 to 8 carbon atoms and more preferentially from 1 to 4 carbon atoms, polyols, polyethylene glycols, aromatic alcohols and their mixtures.

Mention may in particular be made, as examples of organic solvents which can be used according to the invention, of ethanol, propanol, butanol, isopropanol, isobutanol, propylene glycol, dipropylene glycol, isoprene glycol, butylene glycol, glycerol, sorbitol, benzyl alcohol, phenoxyethanol and their mixtures.

Preferably, the organic solvent(s) are chosen from linear or branched monoalcohols having from 1 to 4 carbon atoms and their mixtures, and more preferentially from ethanol, propanol, butanol, isopropanol, isobutanol and their mixtures, and better still the organic solvent is ethanol.

The total amount of the organic solvent(s) present in the cosmetic composition contained in the aerosol device according to the invention is preferably greater than or equal to 5% by weight and more preferentially ranges from 5% to 15% by weight, with respect to the total weight of the composition.

Preferably, the composition according to the invention comprises an amount of water of less than 5% by weight, preferentially of less than 2% by weight and better still of less than 1% by weight, with respect to the total weight of the composition.

### The propellants

The cosmetic composition contained in the aerosol device according to the present invention additionally comprises one or more propellants, at least one of which is dimethyl ether.

In other words, the cosmetic composition contained in the aerosol device according to the invention comprises dimethyl ether and optionally one or more additional propellants other than dimethyl ether.

The additional propellant(s) other than dimethyl ether which can be used according to the invention are preferably chosen from liquefied gases and their mixtures, more preferentially from chlorinated and/or fluorinated hydrocarbons, such as trichlorofluoromethane, dichlorodifluoromethane, chlorodifluoromethane, 1,1,1,2-tetrafluoroethane, chloropentafluoroethane, 1-chloro-1,1-difluoroethane or 1,1-difluoroethane; volatile hydrocarbons, which are optionally halogenated, such as, in particular, C₃ to C₅ alkanes, such as propane, isopropane, n-butane, isobutane, pentane and their mixtures, more preferentially from C₃ to C₅ alkanes and their mixtures, and better still from propane, isopropane, n-butane, isobutane and their mixtures.

When the cosmetic composition contained in the aerosol device comprises one or more additional propellant(s) other than dimethyl ether, the ratio by weight of the total amount of dimethyl ether to the total amount of the additional propellant(s) other than dimethyl ether preferably ranges from 0.5 to 2, more preferentially from 0.8 to 1.5 and better still is equal to 1.

The amount of dimethyl ether present in the cosmetic composition contained in the aerosol device according to the invention is preferably greater than or equal to 30% by weight, more preferentially ranges from 30% to 90% by weight and better still from 35% to 85% by weight, with respect to the total weight of the composition.

The total amount of the propellant(s), which are present in the cosmetic composition contained in the aerosol device according to the invention (i.e. corresponding to the total amount of all of the propellants present in the cosmetic composition, that is to say dimethyl ether and optionally one or more additional propellants, is preferably greater than or equal to 65% by weight, more preferentially greater than or equal to 70% by weight and better still ranges from 75% to 85% by weight, with respect to the total weight of the composition.

### Additives

The cosmetic composition contained in the aerosol device according to the present invention can optionally also comprise one or more additives, other than the compounds of the invention, and among which mention may be made of cationic, anionic, non-ionic, amphoteric or zwitterionic surfactants, and their mixtures, non-silicone fatty substances, cationic, anionic, non-ionic or amphoteric polymers, or their mixtures, other than the fixing polymers of the invention, antidandruff agents, anti-seborrhoeic agents, vitamins and provitamins, including panthenol, sunscreens, sequestering agents, plasticizing agents, solubilizing agents, acidifying agents, mineral or organic thickening agents, in particular polymeric thickening agents, antioxidants, hydroxy acids, fragrances and preservatives.

Of course, a person skilled in the art will take care to choose this or these optional additional compound(s) so that the advantageous properties intrinsically attached to the composition according to the invention are not, or not substantially, detrimentally affected by the envisaged addition(s).

The above additives can generally be present in an amount, for each of them, of between 0% and 20% by weight, with respect to the total weight of the composition.

### Aerosol device

The aerosol device comprising the cosmetic composition according to the present invention is constituted of a container containing the said composition and of a means for spraying the said composition.

The composition according to the invention is advantageously packaged under pressure, in an aerosol device, for example a monobloc device, which comprises a spraying means and a container.

The spraying means is generally constituted of a dispensing valve controlled by a dispensing head, itself comprising a nozzle through which the composition of the invention is sprayed.

The container containing the pressurized composition can be opaque or transparent. It can be made of glass, of polymer or of metal, and can optionally be coated with a protective varnish layer.

Another subject-matter of the invention is a method for the cosmetic treatment of keratinous fibres, in particular human keratinous fibres, such as the hair, comprising a stage of application, to the said keratinous fibres, of a cosmetic composition as defined above and sprayed from an aerosol device as defined above.

The composition according to the invention is generally applied to dry keratinous substances which have optionally been washed with a shampoo.

Preferably, on conclusion of the stage of application of the composition according to the invention, the keratinous fibres are not rinsed.

Another subject-matter of the invention is the use of a cosmetic composition as defined above and sprayed from an aerosol device according to the present invention for the styling and/or the colouring of keratinous fibres, in particular human keratinous fibres, such as the hair, that is to say for the shaping of the fibres and/or the form retention of the hairstyle.

The following examples serve to illustrate the invention without, however, exhibiting a limiting nature.

### Examples

### Example I

### a) Test composition

The following composition (A) according to the invention was prepared from the ingredients, the contents of which are shown in the table below as percentage by weight of active material with respect to the total weight of the composition.

**Table 1]**

| | Composition A (invention) |
|---|---|
| Polyvinylcaprolactam | 2.4 |
| Calcium carbonate | 2.94 |
| Disteardimonium hectorite | 0.5 |
| Mica | 1.45 |
| Titanium dioxide | 1 |
| Carmine | 0.05 |
| Dimethiconol | 0.13 |
| Dimethicone | 0.98 |
| Dimethyl ether | 40 |
| Butane | 40 |
| Fragrance | 0.2 |
| Ethanol | Q.s. for 100 |

### b) Procedure

100 grams of composition (A) according to the invention are introduced into an aerosol can equipped with a powder valve and with a direct outlet actuator with a 0.5 mm nozzle. After crimping on the valve, the aerosol device is pressurized.
Composition (A) is subsequently sprayed over locks of hair in the proportion of 3 g of composition per lock of hair.

### c) Results

The composition according to the invention results in a good colouring of the locks, while conferring fixing, manageability and bestowal of body on them.
Furthermore, no transfer of the colouring onto the fingers is observed when passing the fingers along the dry locks.

### Example II

### a) Test composition

Composition (B) according to the invention was prepared from the ingredients, the contents of which are shown in the table below as percentage by weight of active material with respect to the total weight of the composition.

**[Table 2]**

| | Composition B (invention) |
|---|---|
| Polyvinylcaprolactam | 2.4 |
| Calcium carbonate | 2.94 |
| Disteardimonium hectorite | 0.5 |
| Dimethiconol | 0.13 |
| Dimethicone | 0.98 |
| Dimethyl ether | 40 |
| Butane | 40 |
| Fragrance | 0.2 |
| Ethanol | Q.s. for 100 |

### b) Procedure

100 grams of composition (B) according to the invention are introduced into an aerosol can equipped with a powder valve and with a direct outlet actuator with a 0.5 mm nozzle. After crimping on the valve, the aerosol device is pressurized.
Composition (B) is subsequently sprayed for 2 seconds at a distance of 20 cm over locks of hair washed and rinsed beforehand.
After a leave-in time of one hour, the locks of hair obtained above were attached to the sample carrier of a Zwick 1120 testing machine (Zwick, Germany) which makes it possible to measure the resistance of the lock to bending, representative of the power of the composition to bestow body. A bending rod is lowered, imposing a pressure on the lock. A force sensor records the force necessary for the bending of the lock. The maximum bending force is thus determined for each of the locks.
The higher the value of the force applied, the greater the bestowal of body on the locks, that is to say that they exhibit more texture. Conversely, the lower the value of the force applied, the lower the bestowal of body on the locks.
The evaluation of the bestowal of body was evaluated for 10 locks of hair before and after application of composition (B).

### c) Results

The maximum force values obtained for composition (B), before and after application, are given in the table below (mean for 10 locks).

**[Table 3]**

| | Force (N) |
|---|---|
| Before application | 119.27 |
| After application | 127.05 |
| p-value (Student's test) | 0.002 |

The results obtained above show that the resistance of the lock to bending is greater after treatment: this illustrates the body-bestowing performance qualities conferred by composition B of the invention. This is because, after application, the hair is significantly more embodied and texturized.

### Example III

### a) Test composition

Composition (B) according to the invention was prepared from the ingredients, the contents of which are shown in the table of Example II.

### b) Procedure

100 grams of composition (B) according to the invention are introduced into an aerosol can equipped with a powder valve and with a direct outlet actuator with a 0.5 mm nozzle. After crimping on the valve, the aerosol device is pressurized.

Composition (B) is subsequently sprayed for 2 seconds at a distance of 20 cm over locks of hair washed and rinsed beforehand.

After a leave-in time of one hour, the locks of hair obtained above were suspended in a measurement device equipped with a CCD (Charge Coupled Device) camera and provided with a regular system for rotation of the lock.

The camera collects, for each lock, 10 images of shadows left by the locks, corresponding to 10 different angles of rotation between 0° and 180°. The images are subsequently processed by image analysis: the area of each shadow is determined using software.

The volume is directly related to the mean of the areas which are measured at the different angles.

The greater the mean area of the surfaces of shadows, the greater the volume of the lock.

The evaluation of the volume was carried out on 5 locks which have been treated with composition (B) and 5 untreated locks.

### c) Results

The means of the areas of the surfaces corresponding, on the one hand, to the 5 locks treated with composition (B) and, on the other hand, to the 5 untreated locks are given in the table below:

**[Table 4]**

| | Area (cm²) |
|---|---|
| Without treatment with composition (B) | 63.13 |
| After application of composition (B) | 69.96 |
| p-value (Student's test) | < 0.001 |

The results obtained above show that the volume of the locks is significantly greater after treatment with composition (B) of the invention: this shows the performance qualities for improvement in the volume which are conferred by composition B. This is because, after application, the hair has significantly greater volume.

### Example IV

### a) Test composition

Compositions (C) (composition according to the invention) and (D) (comparative composition) were prepared from the ingredients, the contents of which are shown in the table below as percentage by weight of active material with respect to the total weight of the composition.

**[Table 5]**

| | Composition C (invention) | Composition D (comparative) |
|---|---|---|
| Polyvinylcaprolactam | 2.4 | 2.4 |
| Calcium carbonate | 3 | 3 |
| Iron oxides (and) triethoxycaprylylsilane | 2 | 2 |
| Disteardimonium hectorite | 0.5 | 0.5 |
| Dimethiconol | 0.18 | 0.18 |
| Dimethicone | 1.32 | 1.32 |
| Dimethyl ether | 45 | - |
| Butane | 35 | 80 |
| Fragrance | 0.2 | 0.2 |
| Ethanol | Q.s. for 100 | Q.s. for 100 |

Compositions (C) and (D) are packaged in transparent aerosol containers.

It is observed that the comparative composition (D) containing only a hydrocarbon propellant (butane) precipitates, unlike the composition of the invention (C) comprising a DME/butane mixture.

The presence of DME makes it possible in particular to prevent problems of blockage.

## Claims

1. Aerosol device containing a cosmetic composition comprising:
- one or more fixing polymers,
- one or more powders,
- at least 0.05% by weight, with respect to the total weight of the composition, of one or more polydimethylsiloxanes comprising dimethylsilanol end groups,
- one or more organic solvents, and
- one or more propellants, at least one of which is dimethyl ether;
the ratio by weight of the total amount of the powder(s) to the total amount of the fixing polymer(s) is greater than or equal to 1.

2. Device according to Claim 1, **characterized in that** the fixing polymer(s) are chosen from anionic fixing polymers, cationic fixing polymers, amphoteric fixing polymers, non-ionic fixing polymers and their mixtures.

3. Device according to either one of the preceding claims, **characterized in that** the fixing polymer(s) are chosen from non-ionic fixing polymers and their mixtures, preferably from polyalkyloxazolines, vinyl acetate homopolymers, vinyl acetate copolymers, acrylic ester homopolymers and copolymers, copolymers of acrylonitrile and of a non-ionic monomer, styrene homopolymers, styrene copolymers, polyamides, vinyllactam homopolymers, vinyllactam copolymers and their mixtures, and more preferentially from vinyllactam homopolymers and their mixtures.

4. Device according to any one of the preceding claims, **characterized in that** the total amount of the fixing polymer(s) ranges from 0.1% to 15% by weight, preferably from 0.5% to 10% by weight and better still from 1% to 8% by weight, with respect to the total weight of the composition.

5. Device according to any one of the preceding claims, **characterized in that** the powder(s) are chosen from pigments, fillers and their mixtures, preferably from pigments, styling powders and their mixtures, better still from pigments, carbonates and their mixtures, and even better still the powder is calcium carbonate.

6. Device according to any one of the preceding claims, **characterized in that** the total amount of the powder(s) ranges from 0.1% to 15% by weight, preferably from 0.5% to 10% by weight and better still from 1% to 8% by weight, with respect to the total weight of the composition.

7. Device according to any one of the preceding claims, **characterized in that** the ratio by weight of the total amount of the powder(s) to the total amount of the fixing polymer(s) ranges from 1 to 5.

8. Device according to any one of the preceding claims, **characterized in that** the total amount of the polydimethylsiloxane(s) comprising dimethylsilanol end groups is greater than or equal to 0.1% by weight and preferably ranges from 0.1% to 1% by weight, with respect to the total weight of the composition.

9. Device according to any one of the preceding claims, **characterized in that** the ratio by weight of the total amount of the fixing polymer(s) to the total amount of the polydimethylsiloxane(s) comprising dimethylsilanol end groups is greater than or equal to 1, preferably greater than or equal to 5, more preferentially ranges from 5 to 25 and better still from 10 to 20.

10. Device according to any one of the preceding claims, **characterized in that** the organic solvent(s) are chosen from linear or branched monoalcohols having from 1 to 4 carbon atoms and their mixtures and preferably from ethanol, propanol, butanol, isopropanol, isobutanol and their mixtures, and that the total amount of the organic solvent(s) is greater than or equal to 5% by weight and preferably ranges from 5% to 15% by weight, with respect to the total weight of the composition.

11. Device according to any one of the preceding claims, **characterized in that** the composition additionally comprises one or more propellant(s) other than dimethyl ether, preferably chosen from chlorinated and/or fluorinated hydrocarbons, volatile hydrocarbons, which are optionally halogenated, and their mixtures and more preferentially from C₃ to C₅ alkanes and their mixtures.

12. Device according to any one of the preceding claims, **characterized in that** the total amount of the propellant(s) is greater than or equal to 65% by weight, preferably greater than or equal to 70% by weight and more preferentially ranges from 75% to 85% by weight, with respect to the total weight of the composition.

13. Device according to any one of the preceding claims, **characterized in that** the composition also comprises one or more additional silicones other than the polydimethylsiloxanes comprising dimethylsilanol end groups, the total amount of the silicones present in the said composition preferably being greater than or equal to 0.5% by weight, preferentially greater than or equal to 0.8% by weight and better still ranging from 0.8% to 3% by weight, with respect to the total weight of the composition.

14. Method for the cosmetic treatment of keratinous fibres, in particular human keratinous fibres, such as the hair, comprising a stage of application, to the said keratinous fibres, of a composition sprayed from an aerosol device as defined in any one of the preceding claims.

15. Use of a composition sprayed from an aerosol device according to any one of Claims 1 to 13, for the styling and/or the colouring of keratinous fibres, in particular human keratinous fibres, such as the hair.

## Patentansprüche

1. Aerosolvorrichtung, die eine kosmetische Zusammensetzung enthält, die Folgendes umfasst:
- ein oder mehrere fixierende Polymere,
- ein oder mehrere Pulver,
- mindestens 0,05 Gew.-%, bezogen auf das Gesamtgewicht der Zusammensetzung, eines oder mehrerer Polydimethylsiloxane mit Dimethylsilanol-Endgruppen,
- ein oder mehrere organische Lösungsmittel und
- ein oder mehrere Treibmittel, von denen mindestens eines Dimethylether ist;
wobei das Gewichtsverhältnis der Gesamtmenge des Pulvers bzw. der Pulver zur Gesamtmenge des fixierenden Polymers bzw. der fixierenden Polymere größer oder gleich 1 ist.

2. Vorrichtung nach Anspruch 1, **dadurch gekennzeichnet, dass** das fixierende Polymer bzw. die fixierenden Polymere aus anionischen fixierenden Polymeren, kationischen fixierenden Polymeren, amphoteren fixierenden Polymeren, nichtionischen fixierenden Polymeren und Mischungen davon ausgewählt ist bzw. sind.

3. Vorrichtung nach einem der vorhergehenden Ansprüche, **dadurch gekennzeichnet, dass** das fixierende Polymer bzw. die fixierenden Polymere aus nichtionischen fixierenden Polymeren und Mischungen davon, vorzugsweise aus Polyalkyloxazolinen, Vinylacetat-Homopolymeren, Vinylacetat-Copolymeren, Acrylsäureester-Homopolymeren und -Copolymeren, Copolymeren von Acrylnitril und einem nichtionischen Monomer, Styrol-Homopolymeren, Styrol-Copolymeren, Polyamiden, Vinyllactam-Homopolymeren, Vinyllactam-Copolymeren und Mischungen davon und weiter bevorzugt aus Vinyllactam-Homopolymeren und Mischungen davon ausgewählt ist bzw. sind.

4. Vorrichtung nach einem der vorhergehenden Ansprüche, **dadurch gekennzeichnet, dass** die Gesamtmenge des fixierenden Polymers bzw. der fixierenden Polymere im Bereich von 0,1 bis 15 Gew.-%, vorzugsweise von 0,5 bis 10 Gew.-% und noch besser von 1 bis 8 Gew.-%, bezogen auf das Gesamtgewicht der Zusammensetzung, liegt.

5. Vorrichtung nach einem der vorhergehenden Ansprüche, **dadurch gekennzeichnet, dass** das Pulver bzw. die Pulver aus Pigmenten, Füllstoffen und Mischungen davon, vorzugsweise aus Pigmenten, Styling-Pulvern und Mischungen davon, noch besser aus Pigmenten, Carbonaten und Mischungen davon ausgewählt ist bzw. sind und sogar noch besser das Pulver Calciumcarbonat ist.

6. Vorrichtung nach einem der vorhergehenden Ansprüche, **dadurch gekennzeichnet, dass** die Gesamtmenge des Pulvers bzw. der Pulver im Bereich von 0,1 bis 15 Gew.-%, vorzugsweise von 0,5 bis 10 Gew.-% und noch besser von 1 bis 8 Gew.-%, bezogen auf das Gesamtgewicht der Zusammensetzung, liegt.

7. Vorrichtung nach einem der vorhergehenden Ansprüche, **dadurch gekennzeichnet, dass** das Gewichtsverhältnis der Gesamtmenge des Pulvers bzw. der Pulver zu der Gesamtmenge des fixierenden Polymers bzw. der fixierenden Polymere im Bereich von 1 bis 5 liegt.

8. Vorrichtung nach einem der vorhergehenden Ansprüche, **dadurch gekennzeichnet, dass** die Gesamtmenge des Polydimethylsiloxans bzw. der Polydimethylsiloxane mit Dimethylsilanol-Endgruppen größer oder gleich 0,1 Gew.-% ist und vorzugsweise im Bereich von 0,1 bis 1 Gew.-%, bezogen auf das Gesamtgewicht der Zusammensetzung, liegt.

9. Vorrichtung nach einem der vorhergehenden Ansprüche, **dadurch gekennzeichnet, dass** das Gewichtsverhältnis der Gesamtmenge des fixierenden Polymers bzw. der fixierenden Polymere zur Gesamtmenge des Polydimethylsiloxans bzw. der Polydimethylsiloxane mit Dimethylsilanol-Endgruppen größer oder gleich 1, vorzugsweise größer oder gleich 5, ist und weiter bevorzugt im Bereich von 5 bis 25 und noch besser von 10 bis 20 liegt.

10. Vorrichtung nach einem der vorhergehenden Ansprüche, **dadurch gekennzeichnet, dass** das organische Lösungsmittel bzw. die organischen Lösungsmittel aus linearen oder verzweigten Monoalkoholen mit 1 bis 4 Kohlenstoffatomen und Mischungen davon und vorzugsweise aus Ethanol, Propanol, Butanol, Isopropanol, Isobutanol und Mischungen davon ausgewählt ist bzw. sind und dass die Gesamtmenge des organischen Lösungsmittels bzw. der organischen Lösungsmittel größer oder gleich 5 Gew.-% ist und vorzugsweise im Bereich von 5 bis 15 Gew.-%, bezogen auf das Gesamtgewicht der Zusammensetzung, liegt.

11. Vorrichtung nach einem der vorhergehenden Ansprüche, **dadurch gekennzeichnet, dass** die Zusammensetzung zusätzlich ein oder mehrere andere Treibmittel als Dimethylether umfasst, die vorzugsweise aus chlorierten und/oder fluorierten Kohlenwasserstoffen, flüchtigen Kohlenwasserstoffen, die gegebenenfalls halogeniert sind, und Mischungen davon und besonders bevorzugt aus C₃- bis C₅-Alkanen und Mischungen davon ausgewählt sind.

12. Vorrichtung nach einem der vorhergehenden Ansprüche, **dadurch gekennzeichnet, dass** die Gesamtmenge des Treibmittels bzw. der Treibmittel größer oder gleich 65 Gew.-%, vorzugsweise größer oder gleich 70 Gew.-%, ist und weiter bevorzugt im Bereich von 75 bis 85 Gew.-%, bezogen auf das Gesamtgewicht der Zusammensetzung, liegt.

13. Vorrichtung nach einem der vorhergehenden Ansprüche, **dadurch gekennzeichnet, dass** die Zusammensetzung außerdem ein oder mehrere zusätzliche Silikone umfasst, die von den Polydimethylsiloxanen mit Dimethylsilanol-Endgruppen verschieden sind, wobei die Gesamtmenge der in der Zusammensetzung enthaltenen Silikone vorzugsweise größer oder gleich 0,5 Gew.-%, bevorzugt größer oder gleich 0,8 Gew.-%, ist und noch besser im Bereich von 0,8 bis 3 Gew.-%, bezogen auf das Gesamtgewicht der Zusammensetzung, liegt.

14. Verfahren zur kosmetischen Behandlung von Keratinfasern, insbesondere menschlichen Keratinfasern, wie dem Haar, umfassend einen Schritt des Aufbringens einer Zusammensetzung, die aus einer Aerosolvorrichtung gemäß einem der vorhergehenden Ansprüche gesprüht wird, auf die Keratinfasern.

15. Verwendung einer aus einer Aerosolvorrichtung nach einem der Ansprüche 1 bis 13 gesprühten Zusammensetzung zum Stylen und/oder zum Färben von Keratinfasern, insbesondere menschlichen Keratinfasern, wie dem Haar.

## Revendications

1. Dispositif aérosol contenant une composition cosmétique comprenant :
- un ou plusieurs polymères fixants,
- une ou plusieurs poudres,
- au moins 0,05% en poids, par rapport au poids total de la composition, d'un ou plusieurs polydiméthylsiloxanes à groupements terminaux diméthylsilanol,
- un ou plusieurs solvants organiques, et
- un ou plusieurs agents propulseurs, dont au moins un est le diméthyléther ;
le rapport pondéral entre la quantité totale de la ou des poudres et la quantité totale du ou des polymères fixants étant supérieur ou égal à 1.

2. Dispositif selon la revendication 1, **caractérisé en ce que** le ou les polymères fixants sont choisis parmi les polymères fixants anioniques, les polymères fixants cationiques, les polymères fixants amphotères, les polymères fixants non ioniques, et leurs mélanges.

3. Dispositif selon l'une quelconque des revendications précédentes, **caractérisé en ce que** le ou les polymères fixants sont choisis parmi les polymères fixants non ioniques et leurs mélanges, de préférence parmi les polyalkyloxazolines ; les homopolymères d'acétate de vinyle ; les copolymères d'acétate de vinyle ; les homopolymères et copolymères d'esters acryliques ; les copolymères d'acrylonitrile et d'un monomère non ionique ; les homopolymères de styrène ; les copolymères de styrène ; les polyamides ; les homopolymères de vinyllactame ; les copolymères de vinyllactame ; et leurs mélanges, et plus préférentiellement parmi les homopolymères de vinyllactame et leurs mélanges.

4. Dispositif selon l'une quelconque des revendications précédentes, **caractérisé en ce que** la quantité totale du ou des polymères fixants va de 0 ,1 à 15% en poids, de préférence de 0,5 à 10% en poids, et mieux de 1 à 8% en poids, par rapport au poids total de la composition

5. Dispositif selon l'une quelconque des revendications précédentes, **caractérisé en ce que** la ou les poudres sont choisies parmi les pigments, les charges et leurs mélanges, de préférence parmi les pigments, les poudres coiffantes et leurs mélanges, mieux parmi les pigments, les carbonates et leurs mélanges ; mieux encore la poudre est le carbonate de calcium.

6. Dispositif selon l'une quelconque des revendications précédentes, **caractérisé en ce que** la quantité totale de la ou des poudres va de 0,1 à 15% en poids, et de préférence de 0,5 à 10% en poids, et mieux encore de 1% à 8% en poids, par rapport au poids total de la composition.

7. Dispositif selon l'une quelconque des revendications précédentes, **caractérisé en ce que** le rapport pondéral entre la quantité totale de la ou des poudres et la quantité totale du ou des polymères fixants va de 1 à 5.

8. Dispositif selon l'une quelconque des revendications précédentes, **caractérisé en ce que** la quantité totale du ou des polydiméthylsiloxanes à groupements terminaux diméthylsilanol est supérieure ou égale à 0,1% en poids, et de préférence va de 0,1 à 1% en poids, par rapport au poids totale de la composition.

9. Dispositif selon l'une quelconque des revendications précédentes, **caractérisé en ce que** le rapport pondéral entre la quantité totale du ou des polymères fixants et la quantité totale du ou des polydiméthylsiloxanes à groupements terminaux diméthylsilanol est supérieur ou égal à 1, de préférence supérieur ou égal à 5, plus préférentiellement va de 5 à 25, et mieux encore de 10 à 20.

10. Dispositif selon l'une quelconque des revendications précédentes, **caractérisé en ce que** le ou les solvants organiques sont choisis parmi les monoalcools linéaires ou ramifiés ayant de 1 à 4 atomes de carbone et leurs mélanges, et de préférence parmi l'éthanol, le propanol, le butanol, l'isopropanol, l'isobutanol et leurs mélanges, et **en ce que** la quantité totale du ou des solvants organiques est supérieure ou égale à 5% en poids, et de préférence va de 5 à 15% en poids, par rapport au poids total de la composition.

11. Dispositif selon l'une quelconque des revendications précédentes, **caractérisé en ce que** la composition comprend en outre un ou plusieurs agents propulseurs différents du diméthyléther, de préférence choisis parmi les hydrocarbures chlorés et/ou fluorés, les hydrocarbures volatiles, éventuellement halogénés, et leurs mélanges, et plus préférentiellement parmi les alcanes en C₃ à C₅ et leurs mélanges.

12. Dispositif selon l'une quelconque des revendications précédentes, **caractérisé en ce que** la quantité totale du ou des agents propulseurs est supérieure ou égale à 65% en poids, de préférence supérieure ou égale à 70% en poids, et plus préférentiellement va de 75 à 85% en poids, par rapport au poids total de la composition.

13. Dispositif selon l'une quelconque des revendications précédentes, **caractérisé en ce que** la composition comprend en outre une ou plusieurs silicones additionnelles différentes des polydiméthylsiloxanes à groupements terminaux diméthylsilanol, la quantité totale des silicones présentes dans ladite composition étant de préférence supérieure ou égale à 0,5% en poids, préférentiellement supérieure ou égale à 0,8% en poids, mieux allant de 0,8 à 3% en poids, par rapport au poids total de la composition.

14. Procédé de traitement cosmétique des fibres kératiniques, en particulier des fibres kératiniques humaines telles que les cheveux, comprenant une étape d'application sur lesdites fibres kératiniques d'une composition pulvérisée à partir d'un dispositif aérosol tel que défini dans l'une quelconque des revendications précédentes.

15. Utilisation d'une composition pulvérisée à partir d'un dispositif aérosol selon l'une quelconque des revendications 1 à 13, pour le coiffage et/ou la coloration des fibres kératiniques, en particulier des fibres kératiniques humaines telles que les cheveux.
